# EUROPEAN PATENT APPLICATION

(11) **EP 4 744 673 A1**
(43) Date of publication of application: **20.05.2026**
(21) Application number: 24838498.4
(22) Date of filing: 11.06.2024
(51) Int. Cl.: A61K 35/74, A61K 39/395, A61P 35/00

(54) **PHARMACEUTICAL COMPOSITION AND USE THEREOF**

(30) Priority: 13.07.2023 CN 202310865060; 31.08.2023 CN 202311119303
(71) Applicant: Moon (Guangzhou) Biotech Co., Ltd., Guangzhou, Guangdong 510535 (CN)
(72) Inventor: XIAN, Yibo, Guangzhou, Guangdong 510535 (CN); CHEN, Zhipeng, Guangzhou, Guangdong 510535 (CN); JIANG, Xianzhi, Guangzhou, Guangdong 510535 (CN)
(74) Representative: Herzog IP Patentanwalts GmbH
(86) International application number: PCT/CN2024/098505
(87) International publication number: WO 2025/011240

(57) **Abstract**

Provided are a pharmaceutical composition and a use thereof. A first aspect of this application provides a pharmaceutical composition including a microorganism-derived component. The pharmaceutical composition can achieve the prevention and treatment of various tumors or cancers, including solid tumors, soft tissue tumors, hematologic tumors, glandular tumors, and metastatic tumors. The pharmaceutical composition demonstrates a prominent immunomodulatory or immunostimulatory effect and an intestinal regulatory function.

## Description

### TECHNICAL FIELD

The present disclosure relates to the technical field of microbiology, and particularly relates to a pharmaceutical composition and a use thereof.

### BACKGROUND

The gut, the largest micro-ecological environment in the human body, is involved in various critical physiological processes such as nutrient absorption, energy metabolism, tissue and organ development, immune defense, and endocrine regulation in the human body. Thus, the gut is closely related to human health. In recent decades, leapfrog advancements have been achieved in the field of gut microecology, and the understanding of gut microecology by humans has been continuously deepened. The human gut harbors a vast community of symbiotic microorganisms. The collective genetic information carried by these symbiotic microorganisms is 50 to 100 times greater than the genetic information of the human. The collective genetic information of these symbiotic microorganisms, known as the gut microbiome, is often referred to as the "second genome" of the human. The gut microbiome constitutes the largest and most direct external environment of the human body, and plays an indispensable role in maintaining human health.

In recent years, with the rapid development of molecular biology, genomics, bioinformatics analysis technologies, high-throughput sequencing, and microbial culture techniques, the influence and effect of gut microbiota on intestinal and extraintestinal diseases have become increasingly well-defined. The gut microbiota works much like an organ with metabolic, immune, and endocrine functions. The gut microbiota can affect the human digestive, circulatory, and nervous systems, and is closely associated with the occurrence of various chronic diseases (such as diabetes, hypertension, cardiovascular diseases, and brain diseases) and tumors. Investigating the interrelationships of gut microbiota with human health and diseases not only constitutes significant scientific research, but also holds profound implications and values for clinical diagnosis, treatment, and even translational applications.

PD-1 exhibits a favorable targeting function, and anti-PD-1 antibodies are commonly used for treating tumors in clinical practice. However, when anti-PD-1 antibodies are used in tumor therapies, the intolerance and non-response may occur, which is primarily attributed to alterations in the tumor microenvironment and compromises the therapeutic efficacy. Therefore, it is necessary to develop strategies capable of improving this situation.

Membrane vesicles (MVs) are functional vesicles released by bacteria into the external environment. MVs are structurally similar to liposomes, and typically have a diameter of 20 nm to 400 nm. In addition to a phospholipid bilayer, MV includes a bacterial outer membrane, various proteins, peptidoglycan, lipopolysaccharides, DNA, RNA, etc. The release of MVs is a crucial secretory mechanism in bacteria. MVs play multifaceted roles in physiological activities of bacteria, including growth, survival, communication, and virulence factor delivery. However, MVs derived from gut microbiota require further understanding and exploitation.

### SUMMARY

The present disclosure is intended to solve at least one of the technical problems existing in the prior art. In view of this, the present disclosure provides a pharmaceutical composition.

A first aspect of the present disclosure provides a pharmaceutical composition, including a microorganism-derived component.

In some implementations of the present disclosure, the microorganism-derived component includes a membrane vesicle (MV) isolated from a bacterial strain.

In some implementations of the present disclosure, the MV includes at least one of a lipid, a protein, and a nucleic acid fragment.

In some implementations of the present disclosure, the nucleic acid fragment includes a DNA fragment.

In some implementations of the present disclosure, the microorganism-derived component includes at least one of following (1) to (5): (1) a bacterial strain with an immunostimulatory or immunomodulatory function, (2) a culture of the bacterial strain with the immunostimulatory or immunomodulatory function, (3) a fermentation supernatant of the bacterial strain with the immunostimulatory or immunomodulatory function, (4) a metabolite of the bacterial strain with the immunostimulatory or immunomodulatory function, and (5) a short-chain fatty acid from the bacterial strain with the immunostimulatory or immunomodulatory function.

In some implementations of the present disclosure, the microorganism-derived component includes the MV isolated from the bacterial strain and further includes at least one of following (1) to (5): (1) the bacterial strain with the immunostimulatory or immunomodulatory function, (2) the culture of the bacterial strain with the immunostimulatory or immunomodulatory function, (3) the fermentation supernatant of the bacterial strain with the immunostimulatory or immunomodulatory function, (4) the metabolite of the bacterial strain with the immunostimulatory or immunomodulatory function, and (5) the short-chain fatty acid from the bacterial strain with the immunostimulatory or immunomodulatory function.

In some implementations of the present disclosure, the microorganism-derived component includes at least 50 wt%, 60 wt%, 70 wt%, 80 wt%, 85 wt%, 90 wt%, 95 wt%, 96 wt%, 97 wt%, 98 wt%, or 99 wt% of the MV.

In some implementations of the present disclosure, the microorganism-derived component includes at least 50 v/v%, 60 v/v%, 70 v/v%, 80 v/v%, 85 v/v%, 90 v/v%, 95 v/v%, 96 v/v%, 97 v/v%, 98 v/v%, or 99 v/v% of the MV.

In some implementations of the present disclosure, the microorganism-derived component includes at least 50 wt%, 60 wt%, 70 wt%, 80 wt%, 85 wt%, 90 wt%, 95 wt%, 96 wt%, 97 wt%, 98 wt%, or 99 wt%, and at least 50 v/v%, 60 v/v%, 70 v/v%, 80 v/v%, 85 v/v%, 90 v/v%, 95 v/v%, 96 v/v%, 97 v/v%, 98 v/v%, or 99 v/v% of the MV.

In some implementations of the present disclosure, the MV is derived from a microbial strain in an intestine of a mammal.

In some implementations of the present disclosure, the MV is derived from a genetically engineered microbial strain in an intestine of a mammal.

In some implementations of the present disclosure, the bacterial strain with the immunostimulatory or immunomodulatory function is any one of following A1) to A6):
A1) a bacterial strain in which 16S rRNA has an identity of at least 95%, 96%, 97%, 98%, 98.5%, 98.65%, 99%, 99.5%, 99.6%, 99.7%, 99.8%, 99.9%, 99.99%, or 100% with a sequence shown in SEQ ID NO: 1;
A2) a bacterial strain with an accession number GDMCC No. 61121;
A3) a bacterial strain in which 16S rRNA has an identity of at least 95%, 96%, 97%, 98%, 98.5%, 98.65%, 99%, 99.5%, 99.6%, 99.7%, 99.8%, 99.9%, 99.99%, or 100% with 16S rRNA of the bacterial strain described in the A2);
A4) a bacterial strain in which a genome has an average nucleotide identity (ANI) of at least 87%, 90%, 95%, 96%, 97%, 98%, 99%, 99.5%, 99.6%, 99.7%, 99.8%, 99.9%, 99.99%, or 100% with a genome of a bacterial strain described in any of the A1) to the A3); and/or, a bacterial strain in which a genome has an alignment fraction of at least 60%, 70%, 80%, 90%, 95%, 96%, 97%, 98%, or 99% with a genome of a bacterial strain described in any of the A1) to the A3);
A5) a bacterial strain of a genus *Enterococcus; and*
A6) at least one bacterial strain of *Enterococcus faecalis (E. faecalis*), *Enterococcus faecium* (*E. faecium*), *Enterococcus asini (E. asini), Enterococcus avium (E. avium), Enterococcus canis (E. canis), Enterococcus casseliflavus (E. casseliflavus*), *Enterococcus cecorum (E. cecorum), Enterococcus columbae (E. columbae), Enterococcus dispar (E. dispar), Enterococcus durans (E. durans), Enterococcus flavescens (E. flavescens), Enterococcus gallinarum (E. gallinarum), Enterococcus gilvus (E. gilvus), Enterococcus haemoperoxidus (E. haemoperoxidus), Enterococcus hirae (E. hirae), Enterococcus malodoratus (E. malodoratus), Enterococcus moraviensis (E. moraviensis), Enterococcus mundtii (E. mundtii), Enterococcus pallens (E. pallens), Enterococcus phoeniculicola (E. phoeniculicola), Enterococcus porcinus (E. porcinus), Enterococcus pseudoavium (E. pseudoavium), Enterococcus raffinosus (E. raffinosus*), *Enterococcus ratti (E. ratti), Enterococcus saccharolyticus (E. saccharolyticus), Enterococcus sulfurous (E. sulfurous*), *Enterococcus villorum (E. villorum), Enterococcus devriesei (E. devriesei), Enterococcus hermanniensis (E. hermanniensis), Enterococcus saccharominimus (E. saccharominimus), Enterococcus aquimarinus (E. aquimarinus), and Enterococcus lactis.*

In some implementations of the present disclosure, the MV is isolated from any bacterial strain of the A1) to the A6);
In some implementations of the present disclosure, a mass percentage and/or volume percentage content of the microorganism-derived component in the pharmaceutical composition is 1% to 99%, 2% to 90%, 5% to 80%, 10% to 70%, 20% to 60%, at least 45%, at least 50%, at least 55%, at least 60%, at least 70%, at least 80%, at least 90%, at least 95%, at least 98%, or at least 99%.

In some implementations of the present disclosure, the pharmaceutical composition further includes at least one of an excipient, a diluent, and a carrier.

In some implementations of the present disclosure, the pharmaceutical composition further includes an adjuvant;
In some implementations of the present disclosure, the adjuvant includes at least one of a lubricant, a wetting agent, a binder, an emulsifier, a suspension stabilizer, a solubilizing agent, a preservative, a sweetener, and a flavoring agent.

In some implementations of the present disclosure, the pharmaceutical composition is a solid preparation or a liquid preparation.

In some implementations of the present disclosure, a dosage form of the pharmaceutical composition is any one of a powder, a granule, a pulvis, a tablet, a capsule, a gel, a suspension, a drop, a drop pill, an injection, a suppository, an aerosol, an oral liquid, an ointment, an emulsion, and an irrigation solution.

In some implementations of the present disclosure, the pharmaceutical composition further includes a package insert.

In some implementations of the present disclosure, the pharmaceutical composition further includes a drug for combination therapy.

In some implementations of the present disclosure, the drug for combination therapy includes an anti-tumor drug.

In some implementations of the present disclosure, the anti-tumor drug includes at least one of a chemotherapeutic drug, a photosensitizer, a photothermal therapy agent, and an immunotherapeutic drug.

In some implementations of the present disclosure, the chemotherapeutic drug includes at least one of paclitaxel, camptothecin, 5-fluorouracil, cisplatin, doxorubicin, mitomycin, and epirubicin.

In some implementations of the present disclosure, the photosensitizer includes at least one of BODIPY, chlorin, and rose bengal.

In some implementations of the present disclosure, the photothermal therapy agent includes at least one of a noble metal nanoparticle, an organic polymer, a carbon-based nanomaterial, a magnetic nanomaterial, and a semiconductor nanomaterial.

In some implementations of the present disclosure, the immunotherapeutic drug includes at least one of an immune cell therapy drug and an immune checkpoint inhibitor.

In some implementations of the present disclosure, the immune cell therapy drug includes at least one of a T cell therapy drug, a tumor-infiltrating lymphocyte therapy drug, and a natural killer (NK) cell therapy drug.

In some implementations of the present disclosure, the immune checkpoint inhibitor includes an inhibitor targeting at least one of PD-1, PD-L1, PD-L2, CTLA-4, TIM-3, LAG-3, TIGIT, VISTA, BTLA, CD27, CD28, CD70, CD80, CD86, CD137, CD276, KIRs, TNFRSF4, GITR, GITRL, 4-1BBL, A2aR, VTCN1, IDO, and KLRA.

A second aspect of the present disclosure provides a use of the above-described pharmaceutical composition in preparation of a drug in at least one of following B1) to B4):
B1) preventing or treating a tumor or cancer;
B2) regulating or stimulating an immune system;
B3) regulating an intestine; and
B4) preventing and/or treating a disease mediated by an STING and/or NOD2 pathway.

In some implementations of the present disclosure, the tumor or cancer in the B1) includes at least one of a solid tumor, a hematologic tumor, a soft tissue tumor, a glandular tumor, and a metastatic tumor.

In some implementations of the present disclosure, the solid tumor includes at least one of melanoma, colorectal cancer, liver cancer, lung cancer, fibrosarcoma, kidney cancer, head and neck cancer, upper gastrointestinal cancer, lower gastrointestinal cancer, hepatobiliary system cancer, neuroendocrine tumor, thoracic cancer, bone tumor, soft tissue sarcoma, skin cancer, breast cancer, female reproductive system cancer, male reproductive system cancer, urinary system cancer, eye cancer, central nervous system cancer, and endocrine system cancer.

In some implementations of the present disclosure, the hematologic tumor includes a tumor in at least one of blood, lymph, and bone marrow.

In some implementations of the present disclosure, the tumor or cancer includes at least one of oral cancer, salivary gland cancer, nasopharyngeal cancer, oropharyngeal cancer, hypopharyngeal cancer, nasal cavity cancer, paranasal sinus cancer, laryngeal cancer, esophageal cancer, gastroesophageal junction cancer, gastric cancer, small intestine cancer, appendix cancer, colon cancer, rectal cancer, anal canal cancer, liver cancer, intrahepatic cholangiocarcinoma, gallbladder cancer, hilar cholangiocarcinoma, distal cholangiocarcinoma, ampullary cancer, pancreatic cancer, gastric neuroendocrine tumor, duodenal and ampullary neuroendocrine tumor, jejuno-ileal neuroendocrine tumor, appendiceal neuroendocrine tumor, colorectal neuroendocrine tumor, pancreatic neuroendocrine tumor, thymic carcinoma, lung cancer, malignant pleural mesothelioma, head and neck sarcomas, soft tissue sarcomas of the trunk and extremities, soft tissue sarcomas of abdominal and thoracic viscera, gastrointestinal stromal tumor, retroperitoneal soft tissue sarcoma, Merkel cell carcinoma, cutaneous malignant melanoma, vulvar cancer, vaginal cancer, cervical cancer, endometrial carcinoma, uterine carcinosarcoma, uterine sarcoma, ovarian cancer, fallopian tube cancer, primary peritoneal cancer, gestational trophoblastic neoplasia, penile cancer, prostate cancer, testicular cancer, kidney cancer, renal pelvis cancer, ureteral cancer, bladder cancer, urethral cancer, eyelid carcinoma, conjunctival carcinoma, conjunctival melanoma, uveal melanoma, retinoblastoma, lacrimal gland cancer, orbital sarcoma, ocular adnexal lymphoma, brain cancer, spinal cord tumor, differentiated thyroid cancer, anaplastic thyroid cancer, medullary thyroid cancer, parathyroid cancer, adrenocortical carcinoma, Hodgkin lymphoma, non-Hodgkin lymphoma, cutaneous lymphoma, plasma cell myeloma, and leukemia.

In some implementations of the present disclosure, the tumor or cancer includes a tumor or cancer caused by a bacterium or virus.

In some implementations of the present disclosure, the bacterium or virus includes at least one of hepatitis B virus, hepatitis C virus, human papillomavirus, Epstein-Barr (EB) virus, *Helicobacter pylori,* and *Fusobacterium nucleatum.*

In some implementations of the present disclosure, the preventing or treating a tumor or cancer is achieved through at least one of following (a) to (j): (a) inhibiting an increase in tumor volume; (b) inhibiting an increase in tumor weight; (c) inhibiting proliferation of a tumor cell; (d) increasing a response rate of a tumor treatment; (e) enhancing therapeutic efficacy of an immunosuppressive drug, including improving therapeutic efficacy of an anti-PD-1 antibody; (f) preventing and/or inhibiting dissemination or metastasis of a tumor cell; (g) reducing resistance to an anti-tumor drug; (h) promoting a transcriptional activity of interferon-β (IFN-β); (i) regulating or activating an immune system of a subject; and (j) improving or activating an activity of the STING and/or NOD2 pathway.

In some implementations of the present disclosure, the regulating or stimulating an immune system in the B2) includes at least one of regulating or stimulating an innate immune response in a gastrointestinal tract, regulating or stimulating an innate immune response outside the gastrointestinal tract, inducing one or more beneficial immune effects in the gastrointestinal tract, and inducing one or more beneficial immune effects outside the gastrointestinal tract.

In some implementations of the present disclosure, the regulating an intestine in the B3) includes at least one of regulating an intestinal function and regulating a gut microbiota.

In some implementations of the present disclosure, the regulating a gut microbiota includes regulating at least one of species, richness, evenness, and diversity of the gut microbiota.

In some implementations of the present disclosure, the regulating a gut microbiota includes regulating a change in at least one of the species, the richness, the evenness, and the diversity of the gut microbiota that is induced by a disease and/or a drug.

In some implementations of the present disclosure, the change in at least one of the species, the richness, the evenness, and the diversity of the gut microbiota that is induced by the disease and/or the drug includes a change in at least one of the species, the richness, the evenness, and the diversity of the gut microbiota that is induced by any one of following D1) to D2): D1) an infectious disease, a tumor or cancer, an inflammation, an allergic disease, or an autoimmune disease; and D2) a drug for preventing and/or treating the D1).

In some implementations of the present disclosure, the disease in the D1) is an intestinal disease.

In some implementations of the present disclosure, the drug in the D2) is an anti-tumor drug.

In some implementations of the present disclosure, the anti-tumor drug in the D2) includes an immunotherapeutic drug.

In some implementations of the present disclosure, the immunotherapeutic drug in the D2) includes an immune checkpoint inhibitor.

In some implementations of the present disclosure, the immune checkpoint inhibitor in the D2) includes an inhibitor targeting at least one of PD-1, PD-L1, PD-L2, CTLA-4, TIM-3, LAG-3, TIGIT, VISTA, BTLA, CD27, CD28, CD70, CD80, CD86, CD137, CD276, KIRs, TNFRSF4, GITR, GITRL, 4-1BBL, A2aR, VTCN1, IDO, and KLRA.

In some implementations of the present disclosure, the disease mediated by the STING and/or NOD2 pathway includes at least one of an infectious disease, a tumor or cancer, an inflammation, an allergic disease, and an autoimmune disease.

A third aspect of the present disclosure provides a use of the above-described pharmaceutical composition in at least one of following B1) to B4):
B1) preventing or treating a tumor or cancer;
B2) regulating or stimulating an immune system;
B3) regulating an intestine; and
B4) preventing and/or treating a disease mediated by an STING and/or NOD2 pathway.

In some implementations of the present disclosure, the tumor or cancer in the B1) includes at least one of a solid tumor, a hematologic tumor, a soft tissue tumor, a glandular tumor, and a metastatic tumor.

In some implementations of the present disclosure, the solid tumor includes at least one of head and neck cancer, upper gastrointestinal cancer, lower gastrointestinal cancer, hepatobiliary system cancer, neuroendocrine tumor, thoracic cancer, bone tumor, soft tissue sarcoma, skin cancer, breast cancer, female reproductive system cancer, male reproductive system cancer, urinary system cancer, eye cancer, central nervous system cancer, and endocrine system cancer.

In some implementations of the present disclosure, the hematologic tumor includes a tumor in at least one of blood, lymph, and bone marrow.

In some implementations of the present disclosure, the tumor or cancer includes at least one of oral cancer, salivary gland cancer, nasopharyngeal cancer, oropharyngeal cancer, hypopharyngeal cancer, nasal cavity cancer, paranasal sinus cancer, laryngeal cancer, esophageal cancer, gastroesophageal junction cancer, gastric cancer, small intestine cancer, appendix cancer, colon cancer, rectal cancer, anal canal cancer, liver cancer, intrahepatic cholangiocarcinoma, gallbladder cancer, hilar cholangiocarcinoma, distal cholangiocarcinoma, ampullary cancer, pancreatic cancer, gastric neuroendocrine tumor, duodenal and ampullary neuroendocrine tumor, jejuno-ileal neuroendocrine tumor, appendiceal neuroendocrine tumor, colorectal neuroendocrine tumor, pancreatic neuroendocrine tumor, thymic carcinoma, lung cancer, malignant pleural mesothelioma, head and neck sarcomas, soft tissue sarcomas of the trunk and extremities, soft tissue sarcomas of abdominal and thoracic viscera, gastrointestinal stromal tumor, retroperitoneal soft tissue sarcoma, Merkel cell carcinoma, cutaneous malignant melanoma, vulvar cancer, vaginal cancer, cervical cancer, endometrial carcinoma, uterine carcinosarcoma, uterine sarcoma, ovarian cancer, fallopian tube cancer, primary peritoneal cancer, gestational trophoblastic neoplasia, penile cancer, prostate cancer, testicular cancer, kidney cancer, renal pelvis cancer, ureteral cancer, bladder cancer, urethral cancer, eyelid carcinoma, conjunctival carcinoma, conjunctival melanoma, uveal melanoma, retinoblastoma, lacrimal gland cancer, orbital sarcoma, ocular adnexal lymphoma, brain cancer, spinal cord tumor, differentiated thyroid cancer, anaplastic thyroid cancer, medullary thyroid cancer, parathyroid cancer, adrenocortical carcinoma, Hodgkin lymphoma, non-Hodgkin lymphoma, cutaneous lymphoma, plasma cell myeloma, and leukemia.

In some implementations of the present disclosure, the tumor or cancer includes a tumor or cancer caused by a bacterium or virus.

In some implementations of the present disclosure, the bacterium or virus includes at least one of hepatitis B virus, hepatitis C virus, human papillomavirus, EB virus, *Helicobacter pylori, and Fusobacterium nucleatum.*

In some implementations of the present disclosure, the preventing or treating a tumor or cancer is achieved through at least one of following (a) to (j): (a) inhibiting an increase in tumor volume; (b) inhibiting an increase in tumor weight; (c) inhibiting proliferation of a tumor cell; (d) increasing a response rate of a tumor treatment; (e) enhancing therapeutic efficacy of an immunosuppressive drug, including improving therapeutic efficacy of an anti-PD-1 antibody; (f) preventing and/or inhibiting dissemination or metastasis of a tumor cell; (g) reducing resistance to an anti-tumor drug; (h) promoting a transcriptional activity of IFN-β; (i) regulating or activating an immune system of a subject; and (j) improving or activating an activity of the STING and/or NOD2 pathway.

In some implementations of the present disclosure, the regulating or stimulating an immune system in the B2) includes at least one of regulating or stimulating an innate immune response in a gastrointestinal tract, regulating or stimulating an innate immune response outside the gastrointestinal tract, inducing one or more beneficial immune effects in the gastrointestinal tract, and inducing one or more beneficial immune effects outside the gastrointestinal tract.

In some implementations of the present disclosure, the regulating an intestine in the B3) includes at least one of regulating an intestinal function and regulating a gut microbiota.

In some implementations of the present disclosure, the regulating a gut microbiota includes regulating at least one of species, richness, evenness, and diversity of the gut microbiota.

In some implementations of the present disclosure, the regulating a gut microbiota includes regulating a change in at least one of the species, the richness, the evenness, and the diversity of the gut microbiota that is induced by a disease and/or a drug.

In some implementations of the present disclosure, the change in at least one of the species, the richness, the evenness, and the diversity of the gut microbiota that is induced by the disease and/or the drug includes a change in at least one of the species, the richness, the evenness, and the diversity of the gut microbiota that is induced by any one of following D1) to D2): D1) an infectious disease, a tumor or cancer, an inflammation, an allergic disease, or an autoimmune disease; and D2) a drug for preventing and/or treating the D1).

In some implementations of the present disclosure, the disease in the D1) is an intestinal disease.

In some implementations of the present disclosure, the drug in the D2) is an anti-tumor drug.

In some implementations of the present disclosure, the anti-tumor drug in the D2) includes an immunotherapeutic drug.

In some implementations of the present disclosure, the immunotherapeutic drug in the D2) includes an immune checkpoint inhibitor.

In some implementations of the present disclosure, the immune checkpoint inhibitor in the D2) includes an inhibitor targeting at least one of PD-1, PD-L1, PD-L2, CTLA-4, TIM-3, LAG-3, TIGIT, VISTA, BTLA, CD27, CD28, CD70, CD80, CD86, CD137, CD276, KIRs, TNFRSF4, GITR, GITRL, 4-1BBL, A2aR, VTCN1, IDO, and KLRA.

In some implementations of the present disclosure, the disease mediated by the STING and/or NOD2 pathway includes at least one of an infectious disease, a tumor or cancer, an inflammation, an allergic disease, and an autoimmune disease.

A fourth aspect of the present disclosure provides a method for preventing or treating a tumor or cancer, and/or regulating or activating an immune system of a subject, and/or regulating an intestine, and/or preventing and/or treating a disease mediated by an STING and/or NOD2 pathway, including administering an effective dose of the above-described pharmaceutical composition to the subject.

In some implementations of the present disclosure, the tumor or cancer includes at least one of a solid tumor, a hematologic tumor, a soft tissue tumor, a glandular tumor, and a metastatic tumor.

In some implementations of the present disclosure, the solid tumor includes at least one of head and neck cancer, upper gastrointestinal cancer, lower gastrointestinal cancer, hepatobiliary system cancer, neuroendocrine tumor, thoracic cancer, bone tumor, soft tissue sarcoma, skin cancer, breast cancer, female reproductive system cancer, male reproductive system cancer, urinary system cancer, eye cancer, central nervous system cancer, and endocrine system cancer.

In some implementations of the present disclosure, the hematologic tumor includes a tumor in at least one of blood, lymph, and bone marrow.

In some implementations of the present disclosure, the tumor or cancer includes at least one of oral cancer, salivary gland cancer, nasopharyngeal cancer, oropharyngeal cancer, hypopharyngeal cancer, nasal cavity cancer, paranasal sinus cancer, laryngeal cancer, esophageal cancer, gastroesophageal junction cancer, gastric cancer, small intestine cancer, appendix cancer, colon cancer, rectal cancer, anal canal cancer, liver cancer, intrahepatic cholangiocarcinoma, gallbladder cancer, hilar cholangiocarcinoma, distal cholangiocarcinoma, ampullary cancer, pancreatic cancer, gastric neuroendocrine tumor, duodenal and ampullary neuroendocrine tumor, jejuno-ileal neuroendocrine tumor, appendiceal neuroendocrine tumor, colorectal neuroendocrine tumor, pancreatic neuroendocrine tumor, thymic carcinoma, lung cancer, malignant pleural mesothelioma, head and neck sarcomas, soft tissue sarcomas of the trunk and extremities, soft tissue sarcomas of abdominal and thoracic viscera, gastrointestinal stromal tumor, retroperitoneal soft tissue sarcoma, Merkel cell carcinoma, cutaneous malignant melanoma, vulvar cancer, vaginal cancer, cervical cancer, endometrial carcinoma, uterine carcinosarcoma, uterine sarcoma, ovarian cancer, fallopian tube cancer, primary peritoneal cancer, gestational trophoblastic neoplasia, penile cancer, prostate cancer, testicular cancer, kidney cancer, renal pelvis cancer, ureteral cancer, bladder cancer, urethral cancer, eyelid carcinoma, conjunctival carcinoma, conjunctival melanoma, uveal melanoma, retinoblastoma, lacrimal gland cancer, orbital sarcoma, ocular adnexal lymphoma, brain cancer, spinal cord tumor, differentiated thyroid cancer, anaplastic thyroid cancer, medullary thyroid cancer, parathyroid cancer, adrenocortical carcinoma, Hodgkin lymphoma, non-Hodgkin lymphoma, cutaneous lymphoma, plasma cell myeloma, and leukemia.

In some implementations of the present disclosure, the tumor or cancer includes a tumor or cancer caused by a bacterium or virus.

In some implementations of the present disclosure, the bacterium or virus includes at least one of hepatitis B virus, hepatitis C virus, human papillomavirus, EB virus, *Helicobacter pylori, and Fusobacterium nucleatum.*

In some implementations of the present disclosure, the preventing or treating a tumor or cancer is achieved through at least one of following (a) to (j): (a) inhibiting an increase in tumor volume; (b) inhibiting an increase in tumor weight; (c) inhibiting proliferation of a tumor cell; (d) increasing a response rate of a tumor treatment; (e) enhancing therapeutic efficacy of an immunosuppressive drug, including improving therapeutic efficacy of an anti-PD-1 antibody; (f) preventing and/or inhibiting dissemination or metastasis of a tumor cell; (g) reducing resistance to an anti-tumor drug; (h) promoting a transcriptional activity of IFN-β; (i) regulating or activating an immune system of a subject; and (j) improving or activating an activity of the STING and/or NOD2 pathway.

In some implementations of the present disclosure, the regulating or stimulating an immune system in the B2) includes at least one of regulating or stimulating an innate immune response in a gastrointestinal tract, regulating or stimulating an innate immune response outside the gastrointestinal tract, inducing one or more beneficial immune effects in the gastrointestinal tract, and inducing one or more beneficial immune effects outside the gastrointestinal tract.

In some implementations of the present disclosure, the regulating an intestine includes at least one of regulating an intestinal function and regulating a gut microbiota.

In some implementations of the present disclosure, the regulating a gut microbiota includes regulating at least one of species, richness, evenness, and diversity of the gut microbiota.

In some implementations of the present disclosure, the regulating a gut microbiota includes regulating a change in at least one of the species, the richness, the evenness, and the diversity of the gut microbiota that is induced by a disease and/or a drug.

In some implementations of the present disclosure, the change in at least one of the species, the richness, the evenness, and the diversity of the gut microbiota that is induced by the disease and/or the drug includes a change in at least one of the species, the richness, the evenness, and the diversity of the gut microbiota that is induced by any one of following D1) to D2): D1) an infectious disease, a tumor or cancer, an inflammation, an allergic disease, or an autoimmune disease; and D2) a drug for preventing and/or treating the D1).

In some implementations of the present disclosure, the disease in the D1) is an intestinal disease.

In some implementations of the present disclosure, the drug in the D2) is an anti-tumor drug.

In some implementations of the present disclosure, the anti-tumor drug in the D2) includes an immunotherapeutic drug.

In some implementations of the present disclosure, the immunotherapeutic drug in the D2) includes an immune checkpoint inhibitor.

In some implementations of the present disclosure, the immune checkpoint inhibitor in the D2) includes an inhibitor targeting at least one of PD-1, PD-L1, PD-L2, CTLA-4, TIM-3, LAG-3, TIGIT, VISTA, BTLA, CD27, CD28, CD70, CD80, CD86, CD137, CD276, KIRs, TNFRSF4, GITR, GITRL, 4-1BBL, A2aR, VTCN1, IDO, and KLRA.

In some implementations of the present disclosure, the disease mediated by the STING and/or NOD2 pathway includes at least one of an infectious disease, a tumor or cancer, an inflammation, an allergic disease, and an autoimmune disease.

In some implementations of the present disclosure, the pharmaceutical composition is administered at least once daily.

In some implementations of the present disclosure, when the pharmaceutical composition is administered twice or more daily, the pharmaceutical composition is administered at a same dose.

In some implementations of the present disclosure, when the pharmaceutical composition is administered twice or more daily, the pharmaceutical composition is administered at different doses.

In some implementations of the present disclosure, a route for the administering includes at least one of oral administration, sublingual administration, nasal administration, rectal administration, inhalation administration, transdermal administration, intragastric administration, intraperitoneal injection, subcutaneous injection, and intramuscular injection.

A fifth aspect of the present disclosure provides a use of C1 and C2 in preparation of an anti-tumor drug, where an anti-tumor activity of the anti-tumor drug includes prevention or treatment of at least one tumor of kidney cancer, liver cancer, and fibrosarcoma;
where, the C1 includes at least one of a bacterial strain of a genus *Enterococcus, a* metabolite of the bacterial strain of the genus *Enterococcus,* a culture of the bacterial strain of the genus *Enterococcus,* and a fermentation supernatant of the bacterial strain of the genus *Enterococcus,* and the C2 includes an immune checkpoint inhibitor;

In some implementations of the present disclosure, the bacterial strain of the genus *Enterococcus* includes at least one of *E. faecalis, E. faecium, E. asini, E. avium, E. canis, E. casseliflavus, E. cecorum, E. columbae, E. dispar, E. durans, E. flavescens, E. gallinarum, E. gilvus, E. haemoperoxidus, E. hirae, E. malodoratus, E. moraviensis, E. mundtii, E. pallens, E. phoeniculicola, E. porcinus, E. pseudoavium, E. raffinosus, E. ratti, E. saccharolyticus, E. sulfurous, E. villorum, E. devriesei, E. hermanniensis, E. saccharominimus, E. aquimarinus,* and *Enterococcus lactis;*

In some implementations of the present disclosure, the bacterial strain of the genus *Enterococcus* is any one of following A1) to A4):
A1) a bacterial strain in which 16S rRNA has an identity of at least 95%, 96%, 97%, 98%, 98.5%, 98.65%, 99%, 99.5%, 99.6%, 99.7%, 99.8%, 99.9%, 99.99%, or 100% with a sequence shown in SEQ ID NO: 1;
A2) a bacterial strain with an accession number GDMCC No. 61121;
A3) a bacterial strain in which 16S rRNA has an identity of at least 95%, 96%, 97%, 98%, 98.5%, 98.65%, 99%, 99.5%, 99.6%, 99.7%, 99.8%, 99.9%, 99.99%, or 100% with 16S rRNA of the bacterial strain described in the A2); and
A4) a bacterial strain in which a genome has an average nucleotide identity (ANI) of at least 87%, 90%, 95%, 96%, 97%, 98%, 99%, 99.5%, 99.6%, 99.7%, 99.8%, 99.9%, 99.99%, or 100% with a genome of a bacterial strain described in any of the A1) to the A3); and/or, a bacterial strain in which a genome has an alignment fraction of at least 60%, 70%, 80%, 90%, 95%, 96%, 97%, 98%, or 99% with a genome of a bacterial strain described in any of the A1) to the A3).

In some implementations of the present disclosure, the immune checkpoint inhibitor includes an inhibitor targeting at least one of PD-1, PD-L1, PD-L2, CTLA-4, TIM-3, LAG-3, TIGIT, VISTA, BTLA, CD27, CD28, CD70, CD80, CD86, CD137, CD276, KIRs, TNFRSF4, GITR, GITRL, 4-1BBL, A2aR, VTCN1, IDO, and KLRA.

In some implementations of the present disclosure, the immune checkpoint inhibitor includes an anti-PD-1 antibody.

A sixth aspect of the present disclosure provides a use of C1 and C2 in prevention or treatment of at least one tumor of kidney cancer, liver cancer, and fibrosarcoma,
where the C1 includes at least one of a bacterial strain of a genus *Enterococcus, a* metabolite of the bacterial strain of the genus *Enterococcus,* a culture of the bacterial strain of the genus *Enterococcus,* and a fermentation supernatant of the bacterial strain of the genus *Enterococcus,* and the C2 includes an immune checkpoint inhibitor.

In some implementations of the present disclosure, the bacterial strain of the genus *Enterococcus* includes at least one of *E. faecalis, E. faecium, E. asini, E. avium, E. canis, E. casseliflavus, E. cecorum, E. columbae, E. dispar, E. durans, E. flavescens, E. gallinarum, E. gilvus, E. haemoperoxidus, E. hirae, E. malodoratus, E. moraviensis, E. mundtii, E. pallens, E. phoeniculicola, E. porcinus, E. pseudoavium, E. raffinosus, E. ratti, E. saccharolyticus, E. sulfurous, E. villorum, E. devriesei, E. hermanniensis, E. saccharominimus, E. aquimarinus,* and *Enterococcus lactis.*

In some implementations of the present disclosure, the bacterial strain of the genus *Enterococcus* is any one of following A1) to A4):
A1) a bacterial strain in which 16S rRNA has an identity of at least 95%, 96%, 97%, 98%, 98.5%, 98.65%, 99%, 99.5%, 99.6%, 99.7%, 99.8%, 99.9%, 99.99%, or 100% with a sequence shown in SEQ ID NO: 1;
A2) a bacterial strain with an accession number GDMCC No. 61121;
A3) a bacterial strain in which 16S rRNA has an identity of at least 95%, 96%, 97%, 98%, 98.5%, 98.65%, 99%, 99.5%, 99.6%, 99.7%, 99.8%, 99.9%, 99.99%, or 100% with 16S rRNA of the bacterial strain described in the A2); and
A4) a bacterial strain in which a genome has an average nucleotide identity (ANI) of at least 87%, 90%, 95%, 96%, 97%, 98%, 99%, 99.5%, 99.6%, 99.7%, 99.8%, 99.9%, 99.99%, or 100% with a genome of a bacterial strain described in any of the A1) to the A3); and/or, a bacterial strain in which a genome has an alignment fraction of at least 60%, 70%, 80%, 90%, 95%, 96%, 97%, 98%, or 99% with a genome of a bacterial strain described in any of the A1) to the A3).

In some implementations of the present disclosure, the immune checkpoint inhibitor includes an inhibitor targeting at least one of PD-1, PD-L1, PD-L2, CTLA-4, TIM-3, LAG-3, TIGIT, VISTA, BTLA, CD27, CD28, CD70, CD80, CD86, CD137, CD276, KIRs, TNFRSF4, GITR, GITRL, 4-1BBL, A2aR, VTCN1, IDO, and KLRA.

In some implementations of the present disclosure, the immune checkpoint inhibitor includes an anti-PD-1 antibody.

A seventh aspect of the present disclosure provides a method for preventing or treating at least one tumor of kidney cancer, liver cancer, and fibrosarcoma, including administering an effective dose of C1 and C2 to a subject,
where the C1 includes at least one of a bacterial strain of a genus *Enterococcus, a* metabolite of the bacterial strain of the genus *Enterococcus,* a culture of the bacterial strain of the genus *Enterococcus,* and a fermentation supernatant of the bacterial strain of the genus *Enterococcus,* and the C2 includes an immune checkpoint inhibitor;

In some implementations of the present disclosure, the bacterial strain of the genus *Enterococcus* includes at least one of *E. faecalis, E. faecium, E. asini, E. avium, E. canis, E. casseliflavus, E. cecorum, E. columbae, E. dispar, E. durans, E. flavescens, E. gallinarum, E. gilvus, E. haemoperoxidus, E. hirae, E. malodoratus, E. moraviensis, E. mundtii, E. pallens, E. phoeniculicola, E. porcinus, E. pseudoavium, E. raffinosus, E. ratti, E. saccharolyticus, E. sulfurous, E. villorum, E. devriesei, E. hermanniensis, E. saccharominimus, E. aquimarinus,* and *Enterococcus lactis;*

In some implementations of the present disclosure, the bacterial strain of the genus *Enterococcus* is any one of following A1) to A4):
A1) a bacterial strain in which 16S rRNA has an identity of at least 95%, 96%, 97%, 98%, 98.5%, 98.65%, 99%, 99.5%, 99.6%, 99.7%, 99.8%, 99.9%, 99.99%, or 100% with a sequence shown in SEQ ID NO: 1;
A2) a bacterial strain with an accession number GDMCC No. 61121;
A3) a bacterial strain in which 16S rRNA has an identity of at least 95%, 96%, 97%, 98%, 98.5%, 98.65%, 99%, 99.5%, 99.6%, 99.7%, 99.8%, 99.9%, 99.99%, or 100% with 16S rRNA of the bacterial strain described in the A2); and
A4) a bacterial strain in which a genome has an average nucleotide identity (ANI) of at least 87%, 90%, 95%, 96%, 97%, 98%, 99%, 99.5%, 99.6%, 99.7%, 99.8%, 99.9%, 99.99%, or 100% with a genome of a bacterial strain described in any of the A1) to the A3); and/or, a bacterial strain in which a genome has an alignment fraction of at least 60%, 70%, 80%, 90%, 95%, 96%, 97%, 98%, or 99% with a genome of a bacterial strain described in any of the A1) to the A3).

In some implementations of the present disclosure, the immune checkpoint inhibitor includes an inhibitor targeting at least one of PD-1, PD-L1, PD-L2, CTLA-4, TIM-3, LAG-3, TIGIT, VISTA, BTLA, CD27, CD28, CD70, CD80, CD86, CD137, CD276, KIRs, TNFRSF4, GITR, GITRL, 4-1BBL, A2aR, VTCN1, IDO, and KLRA.

In some implementations of the present disclosure, the immune checkpoint inhibitor includes an anti-PD-1 antibody.

In some implementations of the present disclosure, the C1 and the C2 are administered at least once daily;
In some implementations of the present disclosure, the C1 and the C2 are administered simultaneously, separately, or sequentially;
In some implementations of the present disclosure, when the C1 or the C2 is administered twice or more daily, the C1 or the C2 is administered at a same dose.

In some implementations of the present disclosure, when the C1 or the C2 is administered twice or more daily, the C1 or the C2 is administered at different doses.

In some implementations of the present disclosure, a route for the administering includes at least one of oral administration, sublingual administration, nasal administration, rectal administration, inhalation administration, transdermal administration, intragastric administration, intraperitoneal injection, subcutaneous injection, and intramuscular injection.

In the embodiments of the present disclosure, MVs, as secretions of the gut bacteria, include a large amount of gut bacterial DNA and substances such as specific proteins. These specific components can activate the immune system of the entire organism, thereby enabling the prevention and treatment of various tumors or cancers, including solid tumors, soft tissue tumors, hematologic tumors, glandular tumors, and metastatic tumors. These specific components demonstrate a prominent immunomodulatory or immunostimulatory effect and even can achieve better outcomes than the direct administration of the gut bacteria. Further, components of the bacterial strain, including a supernatant and an MV of the bacterial strain, can all activate the STING and/or NOD2 pathway. Therefore, when these components of the bacterial strain are prepared into drugs, diverse dosage forms can be adaptively provided based on different needs. Components derived from the bacterial strain, such as the bacterial strain, a metabolite of the bacterial strain, a supernatant of the bacterial strain, and MV secreted by the bacterial strain, can all exert prominent anti-tumor efficacy.

For clinically challenging tumors such as kidney cancer, fibrosarcoma, and liver cancer, achieving effective anti-tumor outcomes with drugs remains highly unpredictable. However, in the embodiments of the present disclosure, combinations of components of *Enterococcus* bacteria with immune checkpoint inhibitors can play a significant therapeutic role. Although these tumor lesions originate outside the gastrointestinal tract, *Enterococcus* bacteria originating from the gut, such as MNC-168, can still be used in combination with immune checkpoint inhibitors to enhance an anti-tumor effect, resulting in improved anti-tumor efficacy. It can be known that the combination of *Enterococcus* bacteria with immune checkpoint inhibitors such as an anti-PD-1 antibody significantly enhances the efficacy of the immune checkpoint inhibitors. Experimental data also reveals that the intervention with the bacterial strain of the present disclosure significantly increases the tumor eradication rate.

In the embodiments of the present disclosure, experiments for the supernatant and MV of the bacterial strain show that the supernatant and MV increase the STING activity 3-fold compared to a control. This may indicate that nucleic acid fragments (such as DNA fragments) present in the supernatant and MV specifically bind to C-Gas, thereby effectively activating the STING and/or NOD2 pathway to exert anti-tumor activity.

In addition, it has been observed that diseases such as cancer, particularly colorectal cancer, are associated with imbalances in the gut microbiota. Even when immunotherapy such as an anti-PD-1 antibody is applied, it remains difficult to substantially ameliorate the imbalances in the gut microbiota, and the diversity of the gut microbiota is still reduced. Such an alteration in the tumor microenvironment may also contribute to intolerance and non-responsiveness during the anti-PD-1 antibody therapy for tumors, ultimately compromising the therapeutic effect. In the present disclosure, the use of the active components of the bacterial strain can make the gut microbiota significantly restored, thereby improving this condition.

Additional aspects and advantages of the present disclosure will be partly provided in the following description, and partly become evident in the following description or can be understood through the practice of the present disclosure.

### BRIEF DESCRIPTION OF THE DRAWINGS

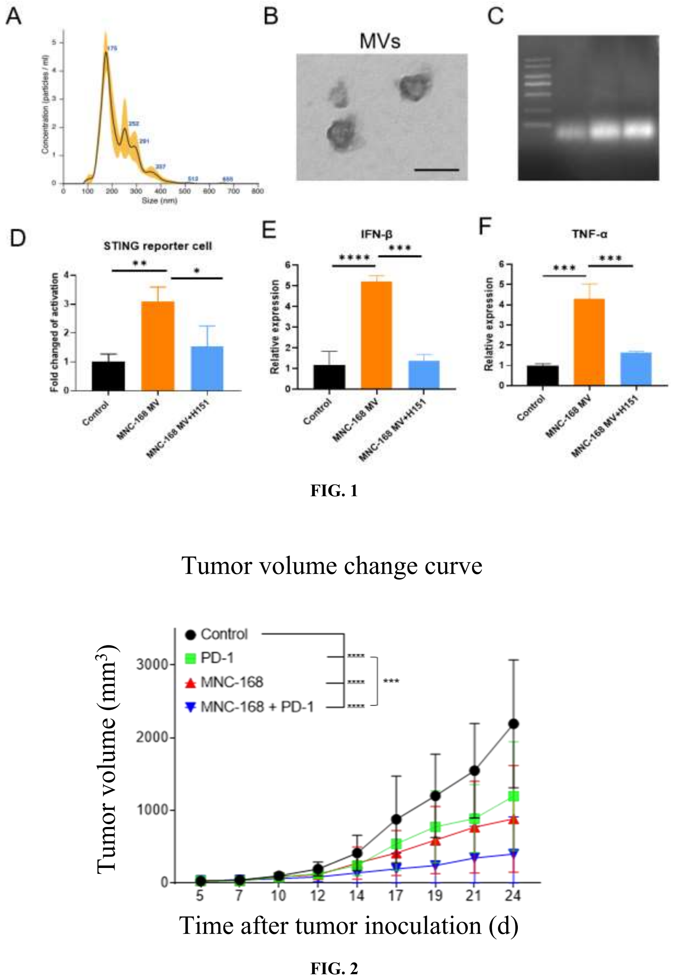

FIG. 1 shows experimental results for *Enterococcus lactis* MNC-168 that activates STING-dependent anti-tumor immunity through MVs in an embodiment of the present disclosure, where A shows a particle size distribution of isolated MVs of *Enterococcus lactis* MNC-168 detected by NanoSight technology; B shows a morphology of the MVs of *Enterococcus lactis* MNC-168 observed by transmission electron microscopy (TEM), with a scale bar of 100 nm; C shows gel electrophoresis-based evaluation results of DNA components in the MVs of *Enterococcus lactis* MNC-168, where a marker is DL2000, and samples in three lanes are 10 µL, 20 µL, and 30 µL of the isolated MVs (MV concentration: 4.5 × 10⁶/µL), respectively; D shows THP-1-STING reporter cell-based assessment results for the activation of an STING activity by the MVs of *Enterococcus lactis* MNC-168, where H-151 (10 µM) is a specific STING inhibitor; E and F show mRNA expression levels of interferon-β (IFN-β) and tumor necrosis factor-α (TNF-α) detected by reverse transcription-polymerase chain reaction (RT-PCR), respectively, where THP-1 cells are treated with the MVs of *Enterococcus lactis* MNC-168 for 24 h and then detected for gene expression, and H-151 (10 µM) is a specific STING inhibitor; and statistical analysis is performed using one-way analysis of variance (ANOVA) in combination with Tukey's test for multiple comparisons;
FIG. 2 shows tumor volume change curves of mice in various groups during intervention processes in an embodiment of the present disclosure, where data is expressed as mean tumor volume ± standard deviation (SD) (mm³), and statistical analysis is performed using two-way ANOVA in combination with Dunnett's test for multiple comparisons;

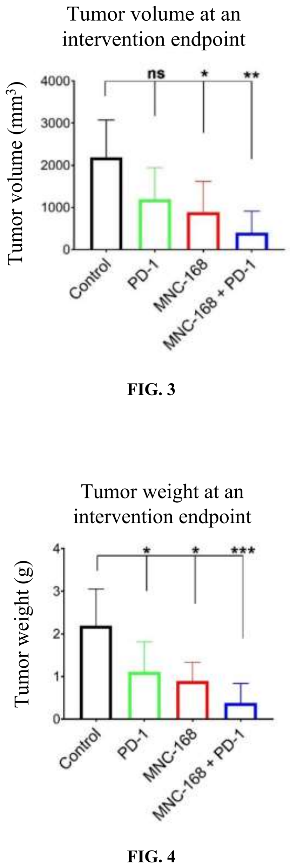

FIG. 3 shows the comparison of tumor volumes of mice in various groups at an intervention endpoint in an embodiment of the present disclosure, where data is expressed as mean tumor volume ± SD (mm³) at the intervention endpoint (D24), and statistical analysis is performed using one-way ANOVA in combination with Tukey's test for multiple comparisons;
FIG. 4 shows the comparison of tumor weights of mice in various groups at an intervention endpoint in an embodiment of the present disclosure, where data is expressed as mean tumor weight ± SD (g) at the intervention endpoint (D24), and statistical analysis is performed using one-way ANOVA in combination with Dunnett's test for multiple comparisons;

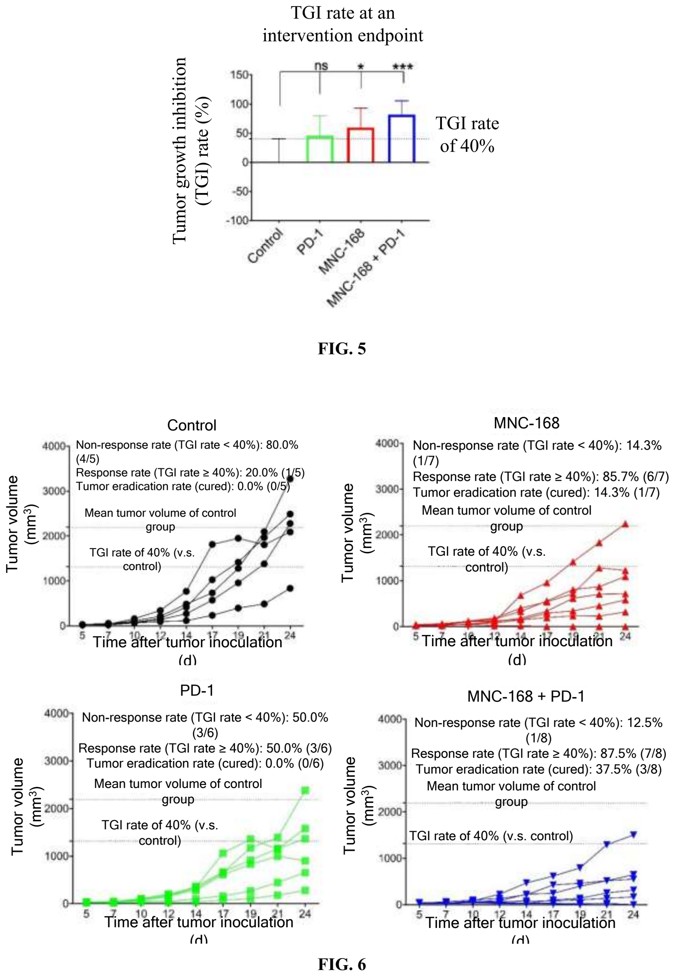

FIG. 5 shows the comparison of tumor growth inhibition (TGI) rates of mice in various groups at an intervention endpoint in an embodiment of the present disclosure, where data is expressed as mean TGI rate ± SD (%); statistical analysis is performed using one-way ANOVA in combination with Dunnett's test for multiple comparisons; a dashed line represents a TGI rate of 40%; and a TGI rate of less than 40% indicates non-responsive anti-tumor efficacy and a TGI rate of more than or equal to 40% indicates responsive anti-tumor efficacy;
FIG. 6 shows tumor volume change curves, anti-tumor response rates, and tumor eradication rates for individual mice in various drug intervention groups in an embodiment of the present disclosure, where this figure shows tumor volume change curves, anti-tumor response rates, and tumor eradication rates for individual mice in a control group, an anti-PD-1 antibody group, an MNC-168 group, and an MNC-168 + anti-PD-1 antibody group; data for individual mice in each drug intervention group is expressed as mean tumor volume ± SD (mm³); an upper dashed line represents a mean tumor volume of the control group (2,193.2 mm³) and a lower dashed line represents a tumor volume (1,315.92 mm³) corresponding to a TGI rate of 40% relative to the control group; when a tumor volume is larger than 1,315.92 mm³, a TGI rate is lower than 40%, indicating non-responsive anti-tumor efficacy; when a tumor volume is smaller than or equal to 1,315.92 mm³, a TGI rate is higher than or equal to 40%, indicating responsive anti-tumor efficacy; when a tumor volume reaches 0 mm³ at an intervention endpoint, it indicates tumor eradication (cured); and calculation formulas are as follows: non-response rate = (number of non-responsive mice/total number of mice in a group) × 100%, response rate = (number of responsive mice/total number of mice in a group) × 100%, and tumor eradication rate = (number of mice with tumor eradication at an endpoint/total number of mice in a group) × 100%;

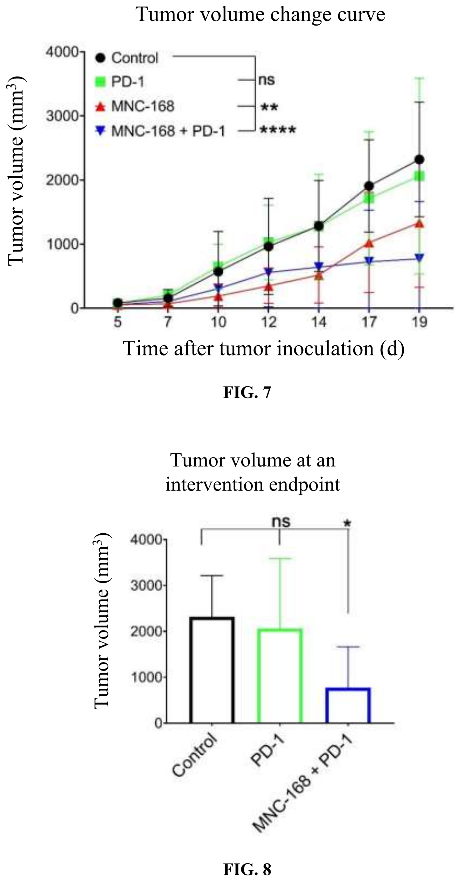

FIG. 7 shows tumor volume change curves of mice in various groups during intervention processes in an embodiment of the present disclosure, where data is expressed as mean tumor volume ± SD (mm³), and statistical analysis is performed using two-way ANOVA in combination with Dunnett's test for multiple comparisons;
FIG. 8 shows the comparison of tumor volumes of mice in various groups at an intervention endpoint in an embodiment of the present disclosure, where data is expressed as mean tumor volume ± SD (mm³) at the intervention endpoint (D19), and statistical analysis is performed using one-way ANOVA in combination with Dunnett's test for multiple comparisons;

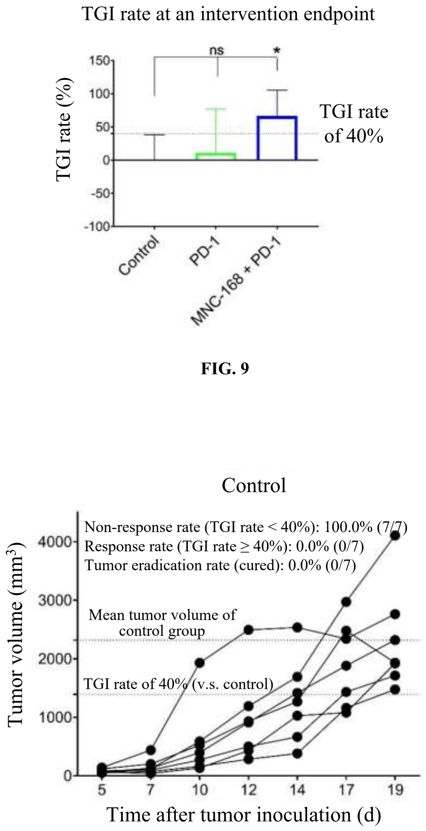

FIG. 9 shows the comparison of TGI rates of mice in various groups at an intervention endpoint in an embodiment of the present disclosure, where data is expressed as mean TGI rate ± SD (%); statistical analysis is performed using one-way ANOVA in combination with Dunnett's test for multiple comparisons; a dashed line represents a TGI rate of 40%; and a TGI rate of less than 40% indicates non-responsive anti-tumor efficacy and a TGI rate of more than or equal to 40% indicates responsive anti-tumor efficacy;

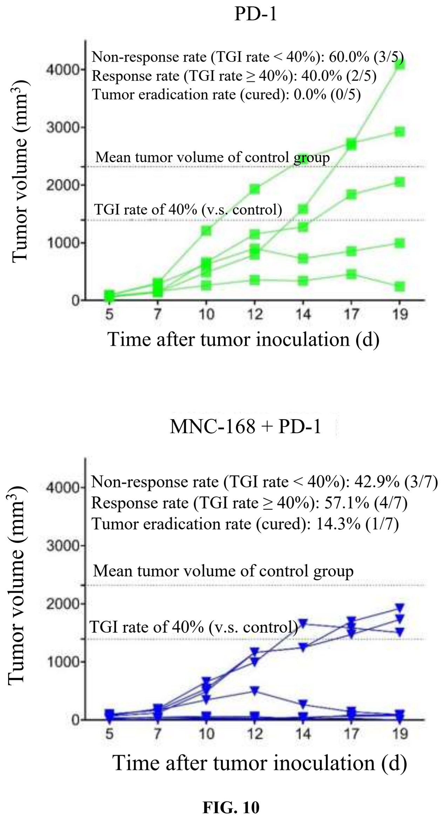

FIG. 10 shows tumor volume change curves, anti-tumor response rates, and tumor eradication rates for individual mice in various drug intervention groups in an embodiment of the present disclosure, where this figure shows tumor volume change curves, anti-tumor response rates, and tumor eradication rates for individual mice in a control group, an anti-PD-1 antibody group, an MNC-168 group, and an MNC-168 + anti-PD-1 antibody group; data for individual mice in each drug intervention group is expressed as mean tumor volume ± SD (mm³); an upper dashed line represents a mean tumor volume of the control group (2,319.43 mm³) and a lower dashed line represents a tumor volume (1,391.658 mm³) corresponding to a TGI rate of 40% relative to the control group; when a tumor volume is larger than 1,391.658 mm³, a TGI rate is lower than 40%, indicating non-responsive anti-tumor efficacy; when a tumor volume is smaller than or equal to 1,391.658 mm³, a TGI rate is higher than or equal to 40%, indicating responsive anti-tumor efficacy; when a tumor volume reaches 0 mm³ at an intervention endpoint, it indicates tumor eradication (cured); and calculation formulas are as follows: non-response rate = (number of non-responsive mice/total number of mice in a group) × 100%, response rate = (number of responsive mice/total number of mice in a group) × 100%, and tumor eradication rate = (number of mice with tumor eradication at an endpoint/total number of mice in a group) × 100%;

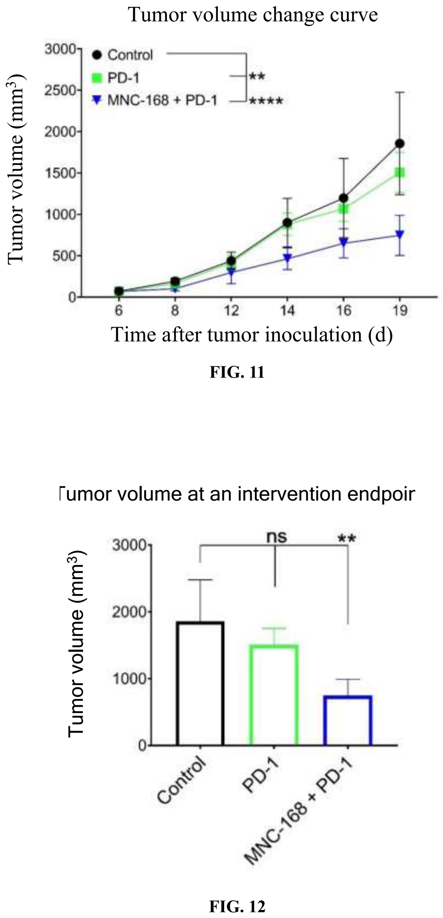

FIG. 11 shows tumor volume change curves of mice in various groups during intervention processes in an embodiment of the present disclosure, where data is expressed as mean tumor volume ± SD (mm³), and statistical analysis is performed using two-way ANOVA in combination with Tukey's test for multiple comparisons;
FIG. 12 shows the comparison of tumor volumes of mice in various groups at an intervention endpoint in an embodiment of the present disclosure, where data is expressed as mean tumor volume ± SD (mm³) at the intervention endpoint (D19), and statistical analysis is performed using one-way ANOVA in combination with Tukey's test for multiple comparisons;

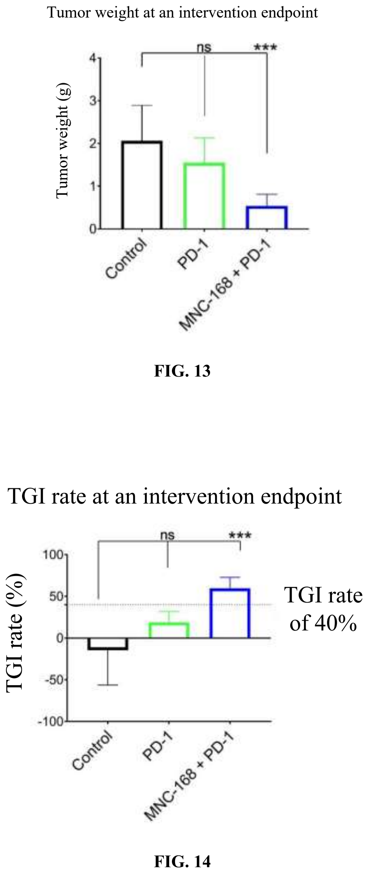

FIG. 13 shows the comparison of tumor weights of mice in various groups at an intervention endpoint in an embodiment of the present disclosure, where data is expressed as mean tumor weight ± SD (g) at the intervention endpoint, and statistical analysis is performed using one-way ANOVA in combination with Tukey's test for multiple comparisons;
FIG. 14 shows the comparison of TGI rates of mice in various groups at an intervention endpoint in an embodiment of the present disclosure, where data is expressed as mean TGI rate ± SD (%); statistical analysis is performed using one-way ANOVA in combination with Tukey's test for multiple comparisons; a dashed line represents a TGI rate of 40%; and a TGI rate of less than 40% indicates non-responsive anti-tumor efficacy and a TGI rate of more than or equal to 40% indicates responsive anti-tumor efficacy;

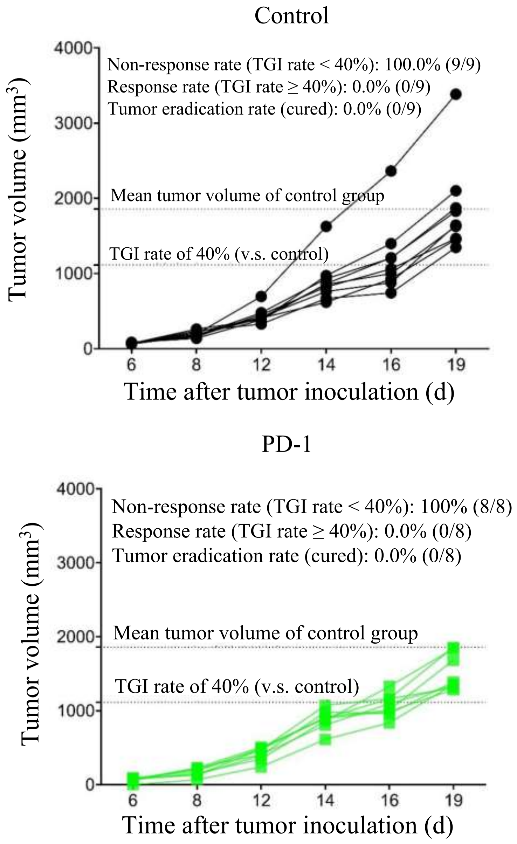

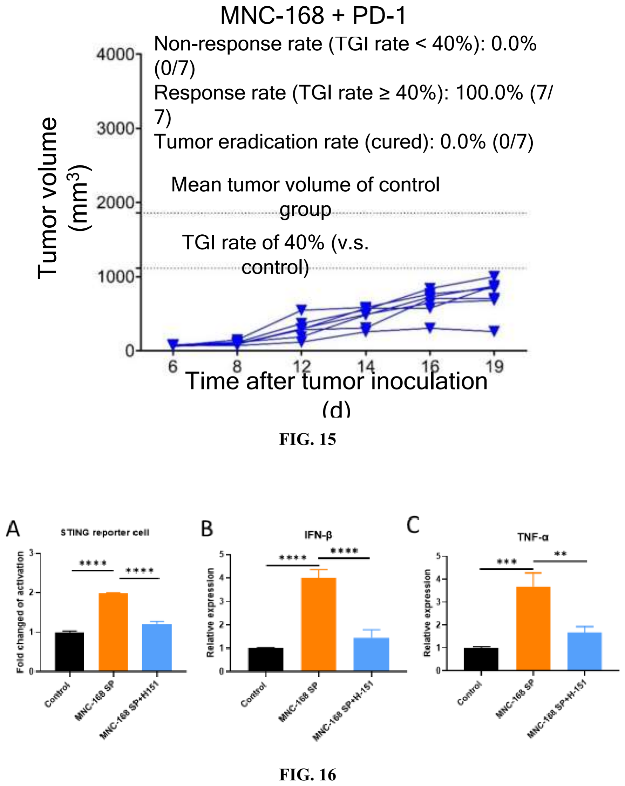

FIG. 15 shows tumor volume change curves, anti-tumor response rates, and tumor eradication rates for individual mice in various drug intervention groups in an embodiment of the present disclosure, where this figure shows tumor volume change curves, anti-tumor response rates, and tumor eradication rates for individual mice in a control group, an anti-PD-1 antibody group, an MNC-168 group, and an MNC-168 + anti-PD-1 antibody group; data for individual mice in each drug intervention group is expressed as mean tumor volume ± SD (mm³); an upper dashed line represents a mean tumor volume of the control group (1,858.33 mm³) and a lower dashed line represents a tumor volume (1,114.998 mm³) corresponding to a TGI rate of 40% relative to the control group; when a tumor volume is larger than 1,114.998 mm³, a TGI rate is lower than 40%, indicating non-responsive anti-tumor efficacy; when a tumor volume is smaller than or equal to 1,114.998 mm³, a TGI rate is higher than or equal to 40%, indicating responsive anti-tumor efficacy; when a tumor volume reaches 0 mm³ at an intervention endpoint, it indicates tumor eradication (cured); and calculation formulas are as follows: non-response rate = (number of non-responsive mice/total number of mice in a group) × 100%, response rate = (number of responsive mice/total number of mice in a group) × 100%, and tumor eradication rate = (number of mice with tumor eradication at an endpoint/total number of mice in a group) × 100%;
FIG. 16 shows experimental results for a supernatant of *Enterococcus lactis* MNC-168 that activates STING-dependent anti-tumor immunity in an embodiment of the present disclosure, where A shows THP-1-STING reporter cell-based assessment results for the activation of an STING activity by the supernatant of *Enterococcus lactis* MNC-168, where H-151 (10 µM) is a specific STING inhibitor; B and C show mRNA expression levels of IFN-β and TNF-α detected by RT-PCR, where THP-1 cells are treated with the supernatant of *Enterococcus lactis* MNC-168 for 24 h and then detected for gene expression, and H-151 (10 µM) is a specific STING inhibitor; and statistical analysis is performed using one-way ANOVA in combination with Tukey's test for multiple comparisons;

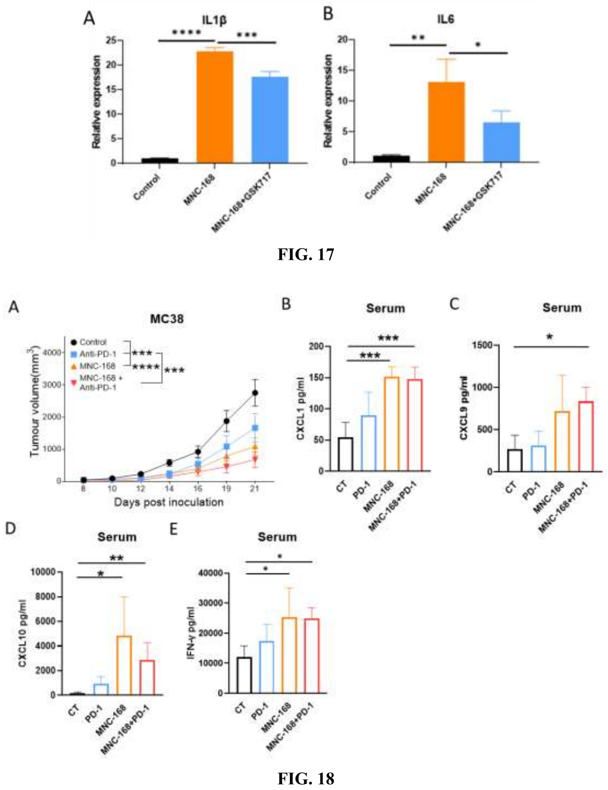

FIG. 17 shows experimental results for a supernatant of *Enterococcus lactis* MNC-168 that activates NOD2-dependent anti-tumor immunity in an embodiment of the present disclosure, where A and B show mRNA expression levels of interleukin-1 β (IL-1β) and interleukin-6 (IL-6) detected by RT-PCR, where THP-1 cells are treated with the supernatant of *Enterococcus lactis* MNC-168 for 24 h and then detected for gene expression, and GSK717 (10 µM) is a specific NOD2 inhibitor; and statistical analysis is performed using one-way ANOVA in combination with Tukey's test for multiple comparisons;
FIG. 18 shows tumor volume change curves of mice in various groups during intervention processes and detection results of corresponding cytokines and chemokines in mouse serum in an embodiment of the present disclosure, where A shows data expressed as mean tumor volume ± SD (mm³), and statistical analysis is performed using two-way ANOVA; and B to E show enzyme-linked immunosorbent assay (ELISA) results for CXCL1, CXCL9, CXCL10, and interferon-γ (IFN-γ), respectively;

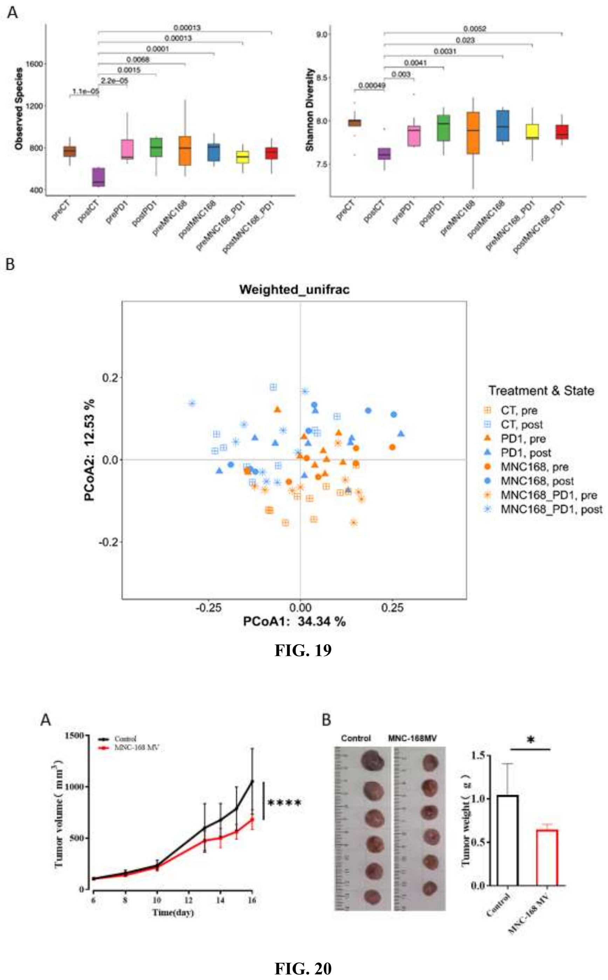

FIG. 19 shows analysis results for gut microbiota diversity in fecal samples from mice of various groups during intervention processes in an embodiment of the present disclosure, where A shows the comparison of gut microbiota species (left panel) and diversity (right panel) among the groups, where statistical analysis is performed using one-way ANOVA in combination with Tukey's test for multiple comparisons; for all intra-group or inter-group comparisons between preintervention and post-intervention, any comparison showing a significant difference is marked with a p value on the graph, and any intra-group comparison not annotated with a p value indicates no significant difference; and B shows principal component analysis (PCA) results for gut microbiota diversity across the groups;
FIG. 20 shows tumor volume change curves of mice in various groups during intervention processes and pictures and weights of tumors at an experimental endpoint in Example 10, where A shows the tumor volume change curves, where data is expressed as mean tumor volume ± SD (mm³), and statistical analysis is performed using two-way ANOVA in combination with Tukey's test for multiple comparisons; and B shows the pictures and weights of tumors at the experimental endpoint, where data is expressed as mean tumor weight ± SD (g) at the experimental endpoint, and statistical analysis is performed using unpaired T-test; and

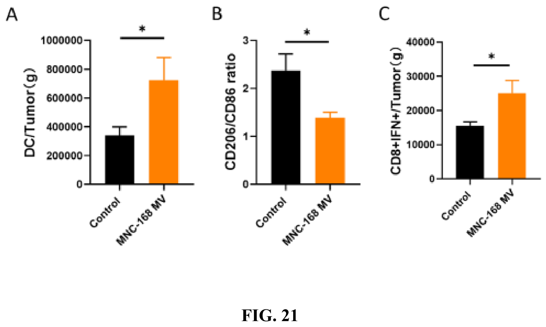

FIG. 21 shows flow cytometry analysis results of immune cell populations from tumor tissues in Example 10, where A shows the comparison of dendritic cell (DC) counts in tumor tissues, B shows a ratio of tumor-associated macrophages (TAMs) expressing CD206 and CD86 in tumor tissues, and C shows the comparison of IFN-γ⁺ CD8⁺ T cell counts in tumor tissues; data is expressed as mean ± SD, and statistical analysis is performed using unpaired T-test; and
a statistical significance is indicated by *: no significant difference (ns): p ≥ 0.05, *: p < 0.05, **: p < 0.01, ***: p < 0.001, and ****: p < 0.0001.

### DETAILED DESCRIPTION OF THE EMBODIMENTS

The concept of the present disclosure and the technical effects produced by the present disclosure will be described clearly and completely below with reference to embodiments, such that the objectives, features, and effects of the present disclosure can be fully understood. Apparently, the described examples are merely some rather than all of the examples of the present disclosure. All other examples obtained by those skilled in the art based on the examples of the present disclosure without creative efforts should fall within the protection scope of the present disclosure.

The embodiments of the present disclosure are described in detail below. The described embodiments are exemplary and only intended to explain the present disclosure, but should not be construed as a limitation to the present disclosure.

In the description of the present disclosure, the term "several" means one or more, the term "a plurality of" means two or more, the terms such as "greater than", "less than", and "exceeding" are construed to exclude the listed number, the terms "no less than", "no more than", and "within" are construed to include the listed number, and the term "about" indicates a range of ±20%, 10%, 8%, 5%, 4%, 3%, 2%, 1%, 0.5%, 0.2%, 0.1%, etc., of the listed number. The "first" and "second" in the description are merely intended to distinguish technical features, rather than to indicate or imply relative importance or implicitly indicate a number of the indicated technical features or implicitly indicate a sequential order of the indicated technical features.

In the description of the present disclosure, the description with reference to the terms "one embodiment", "some embodiments", "exemplary embodiments", "examples", "specific examples", or "some examples" means that specific features, structures, materials, or characteristics described in combination with the embodiments or examples are included in at least one embodiment or example of the present disclosure. In this specification, the schematic representations of the above terms do not necessarily refer to a same embodiment or example. In addition, the specific features, structures, materials, or characteristics described may be combined in any suitable manner in any one or more embodiments or examples.

A first aspect of the embodiments of the present disclosure relates to a pharmaceutical composition, including a microorganism-derived component. The microorganism-derived component includes MV of a bacterium.

In some embodiments, the bacterium corresponding to the MV in the pharmaceutical composition includes a strain *Enterococcus lactis* MNC-168. This strain was deposited in the Guangdong Microbial Culture Collection Center (GDMCC) under an accession number GDMCC No. 61121 on August 05, 2020. The Guangdong Microbial Culture Collection Center (GDMCC) is located at the Guangdong Institute of Microbiology, Guangdong Academy of Sciences on No. 59 Building, No. 100, Xianliezhong Road, Guangzhou. Information on this strain can be seen in WO2022156119A1.

The term "strain" refers to a member of a bacterial species that possesses a genetic characteristic distinguishing the member from closely related members of the same bacterial species. The genetic characteristic may include the complete or partial absence of at least one gene, the complete or partial absence of at least one regulatory region (such as a promoter, a terminator, a riboswitch, and a ribosome binding site), the presence of at least one recombinant gene, the presence of at least one mutated gene, the presence of at least one exogenous gene (a gene from another species), the presence of at least one non-natural plasmid, the presence of at least one antibiotic resistance cassette, or a combination thereof. A genetic characteristic distinguishing between different strains can be identified through polymerase chain reaction (PCR) amplification, and optionally, DNA sequencing can be subsequently conducted for a genomic region of interest or the entire genome. In the case where a strain (compared to another strain of a same species) gains or loses antibiotic resistance, or gains or loses biosynthetic capacity (such as an auxotrophic strain), the bacterial strains can be distinguished through selection or counter-selection using an antibiotic or by meeting a specific nutrition/metabolite requirement.

It is known in the art that strains can be classified and identified through traditional taxonomic approaches and molecular biology techniques. The traditional taxonomic approaches include, for example, cell morphology observation, Gram staining, flagella staining, various metabolic assays, etc. The molecular biology techniques include ribosomal RNA sequencing, whole genome sequencing-based techniques, etc.

Therefore, in some embodiments, in addition to the aforementioned strain MNC-168, the bacterium includes variants of the bacterial strain MNC-168, such as variants possessing at least one identical morphological and/or physio-biochemical identification characteristic to MNC-168.

Further, according to sequencing results for 16S rRNA of the bacterial strain MNC-168, a sequence of the 16S rRNA is shown in SEQ ID NO: 1 (a specific sequence is shown in SEQ ID NO: 1 described in WO2022156119A1). 16S rRNA is a ribosomal RNA in prokaryotes. A 16S rRNA gene includes variable regions and conserved regions. The conserved regions are shared by all bacteria, while the variable regions vary among different bacteria to different degrees. By comparing sequences of 16S rRNA genes in bacteria and calculating evolutionary distances based on nucleotide divergences, a phylogenetic tree can be plotted.

When an identity between 16S rRNA gene sequences of two strains is lower than 98.65%, these two strains can be classified as belonging to different species (refer to Kim, M., Oh, H.-S., Park, S.-C., & Chun, J. (2014). Towards a taxonomic coherence between average nucleotide identity and 16S rRNA gene sequence similarity for species demarcation of prokaryotes. INTERNATIONAL JOURNAL OF SYSTEMATIC AND EVOLUTIONARY MICROBIOLOGY, 64(Pt 2), 346-351, and Liu, C., Du, M.-X., Abuduaini, R., Yu, H.-Y., Li, D.-H., Wang, Y.-J., Liu, S.-J. (2021). Enlightening the taxonomy darkness of human gut microbiomes with a cultured biobank. Microbiome, 9(1), P23; Pablo Yarza, Uniting the classification of cultured and uncultured bacteria and archaea using 16S rRNA gene sequences, Microbiology,volume 12,P635-P645, 2014).

When an identity between 16S rRNA gene sequences of two strains is lower than 95%, these two strains can be classified as belonging to different genera ("A genus definition for Bacteria and Archaea based on genome relatedness and taxonomic affiliation", R.A. Barco, bioRxiv(doi: https://doi.org/10.1101/392480);Evaluation of 16S rRNA gene sequencing for species and strain-level microbiome analysis, Jethro S. Johnson, Nature Communications 10, Article number: 5029 (2019)).

Therefore, in some embodiments of the present disclosure, the bacterium further includes variants of the bacterial strain MNC-168 with the aforementioned 16S rRNA sequence. These variants have an identity of at least 95%, 96%, 97%, 98%, 98.5%, 98.65%, 99%, 99.5%, 99.6%, 99.7%, 99.8%, 99.9%, 99.99%, or 100% with the bacterial strain MNC-168 in terms of 16S rRNA sequences. Consequently, these variants are of the same species as the bacterial strain MNC-168 or of different species from the bacterial strain MNC-168 in a same genus. Thus, it can be reasonably inferred that these strains, either of a same species or different species in a same genus, possess similar biological activities or uses.

The "identity" between sequences of two nucleic acid molecules can be determined using a known computer algorithm, such as at least one of a GCG program package, BLASTN, and FASTA. Other commercial or publicly available programs may include, for example, DNAStar "MegAlign" program.

Moreover, with the rapid development of second-generation and third-generation sequencing technologies, the identification of strains based on whole genome sequencing has become feasible, which can enhance the accuracy of identification results of strains. An average nucleotide identity (ANI) of two bacterial genomes refers to a similarity of homologous genes between the two bacterial genomes. In the field of bacterial taxonomy, it is widely believed that an ANI value of 98.65% or more is required to define two strains as belonging to a same species(Kim, M., Oh, H.-S., Park, S.-C., & Chun, J. (2014). Towards a taxonomic coherence between average nucleotide identity and 16S rRNA gene sequence similarity for species demarcation of prokaryotes. INTERNATIONAL JOURNAL OF SYSTEMATIC AND EVOLUTIONARY MICROBIOLOGY, 64(Pt 2), 346-351, and Liu, C., Du, M.-X., Abuduaini, R., Yu, H.-Y., Li, D.-H., Wang, Y.-J., Liu, S.-J. (2021). Enlightening the taxonomy darkness of human gut microbiomes with a cultured biobank. Microbiome, 9(1), P23; Pablo Yarza, Uniting the classification of cultured and uncultured bacteria and archaea using 16S rRNA gene sequences, Microbiology,volume 12,P635-P645, 2014).

When an identity between 16S rRNA gene sequences of two strains is lower than 95%, these two strains can be classified as belonging to different genera ("A genus definition for Bacteria and Archaea based on genome relatedness and taxonomic affiliation", R.A. Barco, bioRxiv(doi: https://doi.org/10.1101/392480);Evaluation of 16S rRNA gene sequencing for species and strain-level microbiome analysis, Jethro S. Johnson, Nature Communications 10, Article number: 5029 (2019)).

With the above method, those skilled in the art can determine whether a bacterium belongs to the same species as the bacterial strain MNC-168 or is of a different species from the bacterial strain MNC-168 in a same genus. For example, when an isolated strain has an ANI value of at least 87% such as at least 87%, 90%, 95%, 95.5%, 96%, 96.5%, 97%, 97.5%, 98%, 98.5%, 99%, 99.5%, 99.6%, 99.7%, 99.8%, 99.9%, 99.99%, or 100% with the bacterial strain under the accession number GDMCC No. 61121, it can be determined that the isolated strain belongs to the same genus or the same species as the bacterial strain under the accession number GDMCC No. 61121.

There have been various mature calculation tools for ANI, such as the locally operated software Jspecies (/jspecies) and Gegenees (/documentation.html) and the online calculation tools including ANI caculator (http:/enveomics.gatech.edu/), EzGenome (/ezgenome/ani), and ANItools. Therefore, an ANI value between an isolated strain and the bacterial strain MNC-168 can be calculated using any of the aforementioned locally operated software or the online calculation tools and other tools or methods known in the art.

Therefore, in some embodiments of the present disclosure, the bacterium further includes variants of the bacterial strain MNC-168 based on the aforementioned genome. These variants have an ANI value of at least 95%, 96%, 97%, 98%, 98.5%, 98.65%, 99%, 99.5%, 99.6%, 99.7%, 99.8%, 99.9%, 99.99%, or 100% with the bacterial strain MNC-168 in terms of genomes. Consequently, these variants are of the same species as the bacterial strain MNC-168 or of different species from the bacterial strain MNC-168 in a same genus. Thus, it can be reasonably inferred that these strains, either of a same species or different species in a same genus, possess similar biological activities or uses.

In some embodiments, based on the alignment results of both 16S rRNA and ANI, a variant of the bacterial strain carries 16S rRNA having an identity of at least 95%, 96%, 97%, 98%, 98.5%, 98.65%, 99%, 99.5%, 99.6%, 99.7%, 99.8%, 99.9%, 99.99%, or 100% with the 16S rRNA of MNC-168, and carries a genome having an ANI value of at least 87%, 90%, 95%, 96%, 97%, 98%, 99%, 99.5%, 99.6%, 99.7%, 99.8%, 99.9%, 99.99%, or 100% with the genome of MNC-168.

In some embodiments, the bacterium further includes variants of the bacterial strain determined based on the combination of molecular biology identification results with morphological and physio-biochemical identification results. For example, a variant of the bacterial strain carries 16S rRNA having an identity of at least 95%, 96%, 97%, 98%, 98.5%, 98.65%, 99%, 99.5%, 99.6%, 99.7%, 99.8%, 99.9%, 99.99%, or 100% with the 16S rRNA of the bacterial strain MNC-168, and/or carries a genome having an ANI value of at least 87%, 90%, 95%, 96%, 97%, 98%, 99%, 99.5%, 99.6%, 99.7%, 99.8%, 99.9%, 99.99%, or 100% with the genome of the bacterial strain MNC-168. Moreover, the variant possesses at least one identical morphological and/or physio-biochemical identification characteristic to MNC-168.

The term "variant" includes any bacteria derived from the bacterial strain MNC-168, such as bacteria generated through natural mutation or recombination (for example, a mutation resulting from cell proliferation or cell division), resistance screening, or mutation or recombination based on any other mechanism including radiation, a virus, a transposon, or a mutagenic chemical. The variant also includes other strains isolated from natural environments that share the same or similar taxonomic characteristics and properties with the bacterial strain MNC-168. The variant generally includes strains in the genus *Enterococcus* that belong to the same genus as but of different species from MNC-168, or belong to the same species as but exhibit different phenotypic variations from MNC-168.

In some other embodiments, the bacterium further includes other existing species of the genus *Enterococcus,* such as *Enterococcus faecalis (E. faecalis*), *Enterococcus faecium (E. faecium*), *Enterococcus asini (E. asini), Enterococcus avium (E. avium), Enterococcus canis (E. canis), Enterococcus casseliflavus (E. casseliflavus*), *Enterococcus cecorum (E. cecorum), Enterococcus columbae (E. columbae), Enterococcus dispar (E. dispar), Enterococcus durans (E. durans), Enterococcus flavescens (E. flavescens*), *Enterococcus gallinarum (E. gallinarum), Enterococcus gilvus (E. gilvus), Enterococcus haemoperoxidus (E. haemoperoxidus), Enterococcus hirae (E. hirae), Enterococcus malodoratus (E. malodoratus), Enterococcus moraviensis (E. moraviensis), Enterococcus mundtii (E. mundtii), Enterococcus pallens (E. pallens), Enterococcus phoeniculicola (E. phoeniculicola), Enterococcus porcinus (E. porcinus), Enterococcus pseudoavium (E. pseudoavium), Enterococcus raffinosus (E. raffinosus*), *Enterococcus ratti (E. ratti), Enterococcus saccharolyticus (E. saccharolyticus), Enterococcus sulfurous (E. sulfurous*), *Enterococcus villorum (E. villorum), Enterococcus devriesei (E. devriesei), Enterococcus hermanniensis (E. hermanniensis), Enterococcus saccharominimus (E. saccharominimus),* and *Enterococcus aquimarinus (E. aquimarinus).*

The term "microorganism-derived component" refers to a component originating from a microorganism, which may include the intact microorganism, a secretion of the microorganism, a metabolite of the microorganism, cell debris, etc., such as a small molecule (including an amino acid, a nucleoside, a nucleotide, etc.), a macromolecule (including a polypeptide, a carbohydrate, a nucleic acid, a proteoglycan, a lipid, etc.), an extracellular vesicle, and MV. In some embodiments, the microorganism-derived component also includes a culture or a fermentation supernatant of a microorganism.

In some embodiments, a microorganism in the "microorganism-derived component" refers to, for example, the bacterium described above, such as the aforementioned bacterial strain with an immunostimulatory or immunomodulatory function.

In some embodiments, the MV includes the small molecule or the macromolecule mentioned above. For example, the MV includes at least one of a lipid, a protein, and a nucleic acid fragment. The nucleic acid fragment may be, for example, a DNA fragment.

Additionally, considering an origin of the MV, the MV can be derived from a microbial strain in a mammalian gut, or can be derived from a genetically engineered microbial strain.

In some embodiments, a mass percentage (wt%) content of the microorganism-derived component in the pharmaceutical composition is 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99%. In some embodiments, the mass percentage content of the microorganism-derived component in the pharmaceutical composition is 1% to 99%, 2% to 90%, 5% to 80%, 10% to 70%, 20% to 60%, etc. In some embodiments, the mass percentage content of the microorganism-derived component in the pharmaceutical composition is at least 45%, at least 50%, at least 55%, at least 60%, at least 70%, at least 80%, at least 90%, at least 95%, at least 98%, or at least 99%.

In some embodiments, a volume percentage (v/v%) content of the microorganism-derived component in the pharmaceutical composition is 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99%. In some embodiments, the mass percentage content of the microorganism-derived component in the pharmaceutical composition is 1% to 99%, 2% to 90%, 5% to 80%, 10% to 70%, 20% to 60%, etc. In some embodiments, the mass percentage content of the microorganism-derived component in the pharmaceutical composition is at least 45%, at least 50%, at least 55%, at least 60%, at least 70%, at least 80%, at least 90%, at least 95%, at least 98%, or at least 99%.

In some embodiments, the pharmaceutical composition may be a solid preparation or a liquid preparation. Given a specific dosage form to be used, the pharmaceutical composition can be any one of a powder, a granule, a pulvis, a tablet, a capsule, a gel, a suspension, a drop, a drop pill, an injection, a suppository, an aerosol, an oral liquid, an ointment, an emulsion, and an irrigation solution. In some embodiments, the pharmaceutical composition further includes at least one component of an excipient, a diluent, and a carrier to produce the aforementioned dosage forms. Typically, acceptable carriers or diluents are well known to those skilled in the art. For example, at least one of lactose, starch, glucose, sucrose, methyl cellulose, magnesium stearate, mannitol, sorbitol, gum arabic, calcium phosphate, alginate, gum tragacanth, gelatin, calcium silicate, microcrystalline cellulose, polyvinylpyrrolidone, cellulose, syrup, and methyl cellulose may be selected as the carrier or the excipient. At least one of ethanol, glycerol, and water is selected as the diluent. Further, it can be understood that the carrier, the excipient, or the diluent can be selected specifically based on an intended route of administration. For instance, different carriers, excipients, or diluents may be selected specifically for varying dosage forms such as a powder, a granule, a pulvis, a tablet, a capsule, a gel, a suspension, a drop, a drop pill, an injection, a suppository, an aerosol, an oral liquid, an ointment, an emulsion, and an irrigation solution. In addition to the carrier, the excipient, or the diluent, the pharmaceutical composition may include at least one of any suitable lubricant, wetting agent, binder, emulsifier, suspension stabilizer, solubilizing agent, preservative, sweetener, and flavoring agent. The lubricant includes sodium oleate, sodium stearate, magnesium stearate, sodium benzoate, sodium acetate, sodium chloride, talc, mineral oil, etc. The binder includes starch, gelatin, a natural sugar, and a natural or synthetic gum. The natural sugar includes, for example, glucose, anhydrous lactose, free-flow lactose, β-lactose, or a corn sweetener. The natural or synthetic gum includes, for example, gum arabic, gum tragacanth, sodium alginate, carboxymethyl cellulose, or polyethylene glycol. The preservative includes sodium benzoate, sorbic acid, or paraben. The bacterial strain or the culture of the bacterial strain is prepared into any of the aforementioned compositions to achieve the different release requirements of the active ingredient, such as quick release or sustained release.

In some embodiments, the pharmaceutical composition further includes a package insert.

In some embodiments, the pharmaceutical composition further includes a drug for combination therapy. Through the combination therapy, the therapeutic efficacy can be enhanced. In some embodiments, the drug for combination therapy includes an anti-tumor drug. In some embodiments, the anti-tumor drug may include at least one of a chemotherapeutic drug, a photosensitizer, a photothermal therapy agent, and an immunotherapeutic drug.

The chemotherapeutic drug includes at least one of an alkylating agent (such as cyclophosphamide, ifosfamide, thiotepa, melphalan, carmustine, lomustine, semustine, nimustine, fotemustine, estramustine, formylmerphalan, chlormethine, nitrocaphane, nitrogen mustard of phthalic acid, chlorambucil, altretamine, busulfan, and temozolomide), a platinum-based agent (such as cisplatin, carboplatin, oxaliplatin, heptaplatin, lobaplatin, nedaplatin, and dicycloplatin), a podophyllotoxin-based agent (such as podophyllotoxin, etoposide, teniposide, and etoposide phosphate), a camptothecin-based agent (such as camptothecin, hydroxycamptothecin, irinotecan, and topotecan), a taxane (such as paclitaxel and docetaxel), an anthracycline (such as daunorubicin, doxorubicin, epirubicin, pirarubicin, idarubicin, mitoxantrone, and aclarubicin), a fluoropyrimidine (such as gemcitabine, capecitabine, 5-fluorouracil, tegadifur, doxifluridine, tegafur, carmofur, and trifluridine), and an antibiotic (such as mitomycin, bleomycin, and pingyangmycin).

The photosensitizer includes at least one of a porphyrin (such as chlorin, phthalocyanine, and bacteriochlorin), a BODIPY-based agent (such as BODIPY and aza-BODIPY), and rose bengal.

The photothermal therapy agent includes at least one of a noble metal nanoparticle (such as a nanodot, a nanorod, a nanowire, and a nanosheet of gold, silver, platinum, palladium, etc.), an organic polymer (such as polypyrrole, polyaniline, poly(3,4-ethylenedioxythiophene), polystyrene sulfonate, indocyanine green, and a porphyrin), a carbon-based nanomaterial (such as graphene, a carbon nanotube, and a carbonized cellulose product), a magnetic nanomaterial (such as Fe₃O₄), and a semiconductor nanomaterial (such as copper sulfide, molybdenum sulfide, bismuth sulfide, antimony sulfide, gold sulfide, copper selenide, molybdenum selenide, bismuth selenide, antimony selenide, and gold selenide).

The immunotherapeutic drug includes at least one of an immune cell therapy drug and an immune checkpoint inhibitor. The immune cell therapy drug includes at least one of a T cell therapy drug (such as chimeric antigen receptor T-cell therapy (CAR-T) and T-cell receptor T-cell therapy (TCR-T)), a tumor-infiltrating lymphocyte therapy drug, and a natural killer (NK) cell therapy drug. The immune checkpoint inhibitor includes an inhibitor targeting at least one of PD-1, PD-L1, PD-L2, CTLA-4, TIM-3, LAG-3, TIGIT, VISTA, BTLA, CD27, CD28, CD70, CD80, CD86, CD137, CD276, KIRs, TNFRSF4, GITR, GITRL, 4-1BBL, A2aR, VTCN1, IDO, and KLRA.

The term "immune checkpoint inhibitor" refers to an antagonist targeting a corresponding immune checkpoint protein. The immune checkpoint inhibitor can enhance a protein stimulating an immune response or block a protein inhibiting the immune response, thereby exerting an anticancer effect through the immune response. In some embodiments, the immune checkpoint inhibitor may be a protein or peptide, such as a soluble fusion protein. The immune checkpoint inhibitor may be an antibody or an antigen-binding fragment thereof that binds to an immune checkpoint protein to be inhibited, or may be an inhibitory nucleic acid (such as siRNA, shRNA, and antisense RNA) that specifically binds to mRNA encoding the immune checkpoint protein.

In some specific embodiments, the immune checkpoint inhibitor is an antibody, for example, an anti-PD-1 antibody (such as Camrelizumab, Cemiplimab (cemiplimab-rwlc), Dostarlimab, Enlonstobart, Nivolumab, Pembrolizumab, Penpulimab, Prolgolimab, Pucotenlimab, Retifanlimab, Serplulimab, Sintilimab, Tislelizumab, Toripalimab, and Zimberelimab), an anti-PD-L1 antibody (such as Adebrelimab, Atezolizumab, Avelumab, Cosibelimab, Durvalumab, Envafolimab, Socazolimab, Sugemalimab, and Tagitanlimab), and an anti-CTLA-4 antibody (such as Ipilimumab and Tremelimumab).

In some embodiments, the pharmaceutical composition is administered orally. The oral administration may involve swallowing such that the pharmaceutical composition enters the gastrointestinal tract, and/or may be buccal, lingual, or sublingual administration such that the pharmaceutical composition enters the bloodstream directly through the oral cavity. Dosage forms suitable for the oral administration include a solid suppository, a solid microparticle, a semi-solid, and a liquid (including a multiphase or dispersed system), such as a tablet; a soft or hard capsule including multiparticulates or nanoparticles, a liquid (such as an aqueous solution), an emulsion, or a powder; a lozenge (including a liquid filler); a chewable tablet and a gel; a rapidly dispersing dosage form; a suppository; a spray; and a buccal/mucosal adhesive patch.

In some embodiments, the pharmaceutical composition is an enteric preparation, namely, a gastric fluid-resistant preparation (for example, tolerant to gastric pH) suitable for the delivery of the pharmaceutical composition to an intestine through oral administration. When the bacterium or another component of the composition is acid-sensitive, for instance, prone to degradation under gastric conditions, the enteric preparation may be particularly useful.

In some embodiments, the enteric preparation includes an enteric coating. In some embodiments, the enteric preparation is in an enteric-coated dosage form. For example, the enteric preparation may be an enteric tablet or an enteric capsule. The enteric coating may be a conventional enteric coating, such as conventional coatings of tablets, capsules, and similar dosage forms for oral delivery. The enteric preparation may include a film coating, for example, a film layer of an enteric polymer, such as an acid-insoluble polymer. In some embodiments, the enteric preparation is intrinsically enteric, for instance, the enteric preparation is intrinsically gastroresistant without requiring an enteric coating. In some embodiments, the enteric preparation is inherently an enteric capsule (for example, Capsugel^{®}).

In some embodiments, the enteric preparation is a soft capsule. The soft capsule possesses specified elasticity and flexibility due to the presence of a softener such as glycerol, sorbitol, maltitol, and polyethylene glycol in a capsule shell. The soft capsule can be produced on the basis of gelatin or starch, for example. A gelatin-based soft capsule is commercially available from various suppliers. Depending on a route of administration, such as oral or rectal administration, the soft capsule can have various shapes, including, for example, round, oval, oval, or torpedo-shaped soft capsules. The soft capsule can be produced by a conventional process, such as a Scherer process, an Accogel process, or a droplet/blow molding process.

In some embodiments, the enteric preparation is a microencapsulated capsule. In this way, the pharmaceutical composition is protected from degradation through encapsulation until the pharmaceutical composition is delivered to a target site. For instance, the microencapsulated capsule ruptures in response to a chemical or physical stimulus such as pressure, enzymatic activity, or physical decomposition, which may be triggered by a change in pH. Any suitable encapsulation method may be adopted. Exemplary encapsulation techniques include entrapment within a porous matrix, attachment or adsorption on a surface of a solid carrier, self-aggregation through flocculation or the use of a cross-linking agent, and mechanical containment behind a microporous membrane or within a microencapsulated capsule.

In some embodiments, the pharmaceutical composition is in an infant-suitable dosage form, a child-suitable dosage form, or an adult-suitable dosage form, or the pharmaceutical composition is in a dosage form for enteral administration or a dosage form for parenteral administration, or the pharmaceutical composition is an oral preparation or an injection. The bacterial strain in the pharmaceutical composition can at least partially proliferate in an intestine of a subject, and the bacterial strain may be any of a live bacterium (such as an attenuated bacterium or a lyophilized bacterium or an irradiated bacterium) and a dead bacterium.

A second aspect of the embodiments of the present disclosure relates to a use of the pharmaceutical composition in preparation of a drug for preventing or treating a tumor or cancer and/or regulating or stimulating an immune system and/or regulating an intestine and/or preventing and/or treating a disease mediated by an STING and/or NOD2 pathway.

The term "tumor" refers to an abnormal mass or neoplasm in the body resulting from cell division and proliferation exceeding normal levels, while the term "cancer" generally refers to a malignant tumor. In some contexts of the present disclosure, the terms "tumor" and "cancer" may be used interchangeably. The tumor or cancer typically includes a solid tumor, a hematologic tumor, a soft tissue tumor, a glandular tumor, a metastatic tumor, etc. A type of the tumor or cancer includes head and neck cancer, upper gastrointestinal cancer, lower gastrointestinal cancer, hepatobiliary system cancer, neuroendocrine tumor, thoracic cancer, bone tumor, soft tissue sarcoma, skin cancer, breast cancer, female reproductive system cancer, male reproductive system cancer, urinary system cancer, eye cancer, central nervous system cancer, and endocrine system cancer. The hematologic tumor includes a tumor in at least one of blood, lymph, and bone marrow. The head and neck cancer includes oral cancer (such as lip cancer, tongue cancer, floor of mouth cancer, tonsil cancer, and gingival carcinoma), salivary gland cancer, nasopharyngeal cancer, oropharyngeal cancer, hypopharyngeal cancer, nasal cavity cancer, paranasal sinus cancer, laryngeal cancer, etc. The upper gastrointestinal cancer includes esophageal cancer, gastroesophageal junction cancer, gastric cancer, small intestine cancer, etc. The lower gastrointestinal cancer includes appendix cancer, colon cancer, rectal cancer, anal canal cancer, etc. The hepatobiliary system cancer includes liver cancer, intrahepatic cholangiocarcinoma, gallbladder cancer, hilar cholangiocarcinoma, distal cholangiocarcinoma, ampullary cancer, pancreatic cancer, etc. The neuroendocrine tumor includes gastric neuroendocrine tumor, duodenal and ampullary neuroendocrine tumor, jejuno-ileal neuroendocrine tumor, appendiceal neuroendocrine tumor, colorectal neuroendocrine tumor, pancreatic neuroendocrine tumor, etc. The thoracic cancer includes thymic carcinoma, lung cancer (such as small cell lung cancer and non-small cell lung cancer), malignant pleural mesothelioma, etc. The soft tissue sarcoma includes head and neck sarcomas, soft tissue sarcomas of the trunk and extremities, soft tissue sarcomas of abdominal and thoracic viscera, gastrointestinal stromal tumor, and retroperitoneal soft tissue sarcoma. The skin cancer includes Merkel cell carcinoma, cutaneous malignant melanoma, etc. The female reproductive system cancer includes vulvar cancer, vaginal cancer, cervical cancer, endometrial carcinoma, uterine carcinosarcoma, uterine sarcoma, ovarian cancer, fallopian tube cancer, primary peritoneal cancer, gestational trophoblastic neoplasia, etc. The male reproductive system cancer includes penile cancer, prostate cancer, testicular cancer, etc. The urinary system cancer includes kidney cancer, renal pelvis cancer, ureteral cancer, bladder cancer, urethral cancer, etc. The eye cancer includes eyelid carcinoma, conjunctival carcinoma, conjunctival melanoma, uveal melanoma, retinoblastoma, lacrimal gland cancer, orbital sarcoma, ocular adnexal lymphoma, etc. The central nervous system cancer includes brain cancer (such as glioma, meningioma, medulloblastoma, medullary pituitary adenoma, primary central nervous system (CNS) lymphoma, and germ cell tumors of the central nervous system), spinal cord tumor, etc. The endocrine system cancer includes differentiated thyroid cancer, anaplastic thyroid cancer, medullary thyroid cancer, parathyroid cancer, adrenocortical carcinoma, etc. The hematologic tumor includes Hodgkin lymphoma, non-Hodgkin lymphoma (such as diffuse large B-cell lymphoma, follicular lymphoma, mantle cell lymphoma, Burkitt lymphoma, nodal marginal zone lymphoma, and mucosa-associated lymphoid tissue lymphoma), cutaneous lymphoma, plasma cell myeloma, leukemia, etc.

The tumor or cancer also includes a tumor or cancer caused by a bacterium or virus, such as a tumor or cancer caused by at least one bacterium or virus of hepatitis B virus, hepatitis C virus, human papillomavirus, Epstein-Barr (EB) virus, *Helicobacter pylori, and Fusobacterium nucleatum.*

In some embodiments, the pharmaceutical composition prevents or treats the tumor or cancer through at least one of the following (a) to (j): (a) inhibiting an increase in tumor volume; (b) inhibiting an increase in tumor weight; (c) inhibiting proliferation of a tumor cell; (d) increasing a response rate of a tumor treatment; (e) enhancing therapeutic efficacy of an immunosuppressive drug, including improving therapeutic efficacy of an anti-PD-1 antibody; (f) preventing and/or inhibiting dissemination or metastasis of a tumor cell; (g) reducing resistance to an anti-tumor drug; (h) promoting a transcriptional activity of IFN-β; (i) regulating or activating an immune system of a subject; and (j) improving or activating an activity of the STING and/or NOD2 pathway.

In some embodiments, the regulating or stimulating an immune system includes at least one of regulating or stimulating an innate immune response in a gastrointestinal tract, regulating or stimulating an innate immune response outside the gastrointestinal tract, inducing one or more beneficial immune effects in the gastrointestinal tract, and inducing one or more beneficial immune effects outside the gastrointestinal tract.

In some embodiments, the regulating an intestine includes at least one of regulating an intestinal function and regulating a gut microbiota.

The intestine is primarily responsible for the digestion and absorption of proteins, fats, and some carbohydrates in a food and the decomposition of substances such as cellulose through fermentation with moisture and microorganisms in a content of the intestine, thereby producing feces. Therefore, the intestinal function mainly includes the following aspects: (1) movement, such as intestinal peristalsis; (2) secretion, such as an intestinal fluid including hormones, enzymes, etc.; and (3) barrier function, for example, preventing the translocation of bacteria and toxins. Further, as the largest immune organ in the human body, the intestine exhibits an important immune function. Therefore, in some embodiments, the regulating an intestinal function includes modulating at least one of movement, secretion, barrier, and immune functions of the intestine.

The gut microbiota refers to microorganisms present in the intestine. Normally, there is a relatively low microbial load in the duodenum, while there is a relatively high microbial load in the ileum and colon. Based on taxonomic characteristics, the gut microbiota primarily includes *Bacteroidetes* and *Firmicutes,* and also includes *Proteobacteria, Actinobacteria, Fusobacteria, Verrucomicrobia,* and *Cyanobacteria.* According to spatial positions in the intestine, the gut microbiota can also include a mucosa-associated microbiota closely attached to an intestinal mucosa and a luminal microbiota moving freely within an intestinal lumen. Depending on functions, the gut microbiota can include commensal bacteria, opportunistic pathogens, pathogenic bacteria, etc. These gut microbiota species collectively establish the intestinal microecological environment. Consequently, an imbalance in the gut microbiota can lead to various issues in digestive, immune, and nervous systems.

In some embodiments, the regulating a gut microbiota includes regulating at least one of species, richness, evenness, and diversity of the gut microbiota. The species of the gut microbiota refer to species of intestinal microorganisms in the gut microbiota, such as the presence or absence and importance of one or more intestinal microorganisms. The richness of the gut microbiota refers to a number of intestinal microorganisms, for example, a number of intestinal microorganisms at a species level, family, a number of intestinal microorganisms at a genus level, a number of intestinal microorganisms at a family level, and a number of intestinal microorganisms at an order level. The evenness of the gut microbiota refers to disparities among quantities of different intestinal microorganisms. High evenness indicates small disparities among quantities of specific or non-specific intestinal microorganisms, while low evenness indicates large disparities among quantities of specific or non-specific intestinal microorganisms. The diversity of the gut microbiota is a comprehensive index for the richness and evenness, which can be evaluated using at least one of a Simpson's diversity index and a Shannon diversity index, for example.

Typically, the gut microbiota can maintain relatively balanced proportions of microorganisms. However, factors such as diseases and medication may lead to imbalances in the gut microbiota. Thus, in some embodiments, the regulating a gut microbiota includes regulating a change in at least one of the species, the richness, the evenness, and the diversity of the gut microbiota that is induced by a disease and/or a drug, for example, a decline in at least one of the richness, the evenness, and the diversity, a reduction in a quantity of one or more commensal bacteria, or an increase in a quantity of one or more opportunistic pathogens or pathogenic bacteria.

In some embodiments, the change in at least one of the species, the richness, the evenness, and the diversity of the gut microbiota that is induced by the disease and/or the drug includes a change in at least one of the species, the richness, the evenness, and the diversity of the gut microbiota that is induced by any one of the following D1) to D2): D1) an infectious disease, a tumor or cancer, an inflammation, an allergic disease, or an autoimmune disease; and D2) a drug for preventing and/or treating the D1).

In some embodiments, the disease in the D1) is an intestinal disease, for example, at least one of intestinal cancer (including small intestine cancer, colon cancer, rectal cancer, etc.), intestinal infection, intestinal inflammation, and intestinal allergy (such as allergic colitis).

In some embodiments, the drug in the D2) is an anti-tumor drug, which may be an anti-intestinal cancer drug, such as an immunotherapeutic drug including an immune checkpoint inhibitor. Specifically, the immune checkpoint inhibitor includes an inhibitor targeting at least one of PD-1, PD-L1, PD-L2, CTLA-4, TIM-3, LAG-3, TIGIT, VISTA, BTLA, CD27, CD28, CD70, CD80, CD86, CD137, CD276, KIRs, TNFRSF4, GITR, GITRL, 4-1BBL, A2aR, VTCN1, IDO, and KLRA, for example, an anti-PD-1 antibody (such as Camrelizumab, Cemiplimab (cemiplimab-rwlc), Dostarlimab, Enlonstobart, Nivolumab, Pembrolizumab, Penpulimab, Prolgolimab, Pucotenlimab, Retifanlimab, Serplulimab, Sintilimab, Tislelizumab, Toripalimab, and Zimberelimab), an anti-PD-L1 antibody (such as Adebrelimab, Atezolizumab, Avelumab, Cosibelimab, Durvalumab, Envafolimab, Socazolimab, Sugemalimab, and Tagitanlimab), and an anti-CTLA-4 antibody (such as Ipilimumab and Tremelimumab).

In some embodiments, the disease mediated by the STING and/or NOD2 pathway includes at least one of an infectious disease, a tumor or cancer, an inflammation, an allergic disease, and an autoimmune disease.

A third aspect of the embodiments of the present disclosure relates to a use of the pharmaceutical composition in preventing or treating a tumor or cancer and/or regulating or stimulating an immune system and/or regulating an intestine and/or preventing and/or treating a disease mediated by an STING and/or NOD2 pathway.

In some embodiments, the pharmaceutical composition prevents or treats the tumor or cancer through at least one of the following (a) to (j): (a) inhibiting an increase in tumor volume; (b) inhibiting an increase in tumor weight; (c) inhibiting proliferation of a tumor cell; (d) increasing a response rate of a tumor treatment; (e) enhancing therapeutic efficacy of an immunosuppressive drug, including improving therapeutic efficacy of an anti-PD-1 antibody; (f) preventing and/or inhibiting dissemination or metastasis of a tumor cell; (g) reducing resistance to an anti-tumor drug; (h) promoting a transcriptional activity of IFN-β; (i) regulating or activating an immune system of a subject; and (j) improving or activating an activity of the STING and/or NOD2 pathway.

In some embodiments, the regulating or stimulating an immune system includes at least one of regulating or stimulating an innate immune response in a gastrointestinal tract, regulating or stimulating an innate immune response outside the gastrointestinal tract, inducing one or more beneficial immune effects in the gastrointestinal tract, and inducing one or more beneficial immune effects outside the gastrointestinal tract.

In some embodiments, the regulating an intestine includes at least one of regulating an intestinal function and regulating a gut microbiota.

In some embodiments, the regulating a gut microbiota includes regulating at least one of species, richness, evenness, and diversity of the gut microbiota.

In some embodiments, the regulating a gut microbiota includes regulating a change in at least one of the species, the richness, the evenness, and the diversity of the gut microbiota that is induced by a disease and/or a drug.

In some embodiments, the change in at least one of the species, the richness, the evenness, and the diversity of the gut microbiota that is induced by the disease and/or the drug includes a change in at least one of the species, the richness, the evenness, and the diversity of the gut microbiota that is induced by any one of the following D1) to D2): D1) an infectious disease, a tumor or cancer, an inflammation, an allergic disease, or an autoimmune disease; and D2) a drug for preventing and/or treating the D1).

In some embodiments, the disease in the D1) is an intestinal disease.

In some embodiments, the drug in the D2) is an anti-tumor drug.

In some embodiments, the anti-tumor drug in the D2) includes an immunotherapeutic drug.

In some embodiments, the immunotherapeutic drug in the D2) includes an immune checkpoint inhibitor.

In some embodiments, the immune checkpoint inhibitor in the D2) includes an inhibitor targeting at least one of PD-1, PD-L1, PD-L2, CTLA-4, TIM-3, LAG-3, TIGIT, VISTA, BTLA, CD27, CD28, CD70, CD80, CD86, CD137, CD276, KIRs, TNFRSF4, GITR, GITRL, 4-1BBL, A2aR, VTCN1, IDO, and KLRA.

In some embodiments, the disease mediated by the STING and/or NOD2 pathway includes at least one of an infectious disease, a tumor or cancer, an inflammation, an allergic disease, and an autoimmune disease.

A fourth aspect of the embodiments of the present disclosure relates to a method for preventing or treating a tumor or cancer and/or regulating or stimulating an immune system and/or regulating an intestine and/or preventing and/or treating a disease mediated by an STING and/or NOD2 pathway, including administering an effective dose of the pharmaceutical composition to a subject.

In some embodiments, the pharmaceutical composition prevents or treats the tumor or cancer through at least one of the following (a) to (j): (a) inhibiting an increase in tumor volume; (b) inhibiting an increase in tumor weight; (c) inhibiting proliferation of a tumor cell; (d) increasing a response rate of a tumor treatment; (e) enhancing therapeutic efficacy of an immunosuppressive drug, including improving therapeutic efficacy of an anti-PD-1 antibody; (f) preventing and/or inhibiting dissemination or metastasis of a tumor cell; (g) reducing resistance to an anti-tumor drug; (h) promoting a transcriptional activity of IFN-β; (i) regulating or activating an immune system of a subject; and (j) improving or activating an activity of the STING and/or NOD2 pathway.

In some embodiments, the regulating or stimulating an immune system includes at least one of regulating or stimulating an innate immune response in a gastrointestinal tract, regulating or stimulating an innate immune response outside the gastrointestinal tract, inducing one or more beneficial immune effects in the gastrointestinal tract, and inducing one or more beneficial immune effects outside the gastrointestinal tract.

In some embodiments, the regulating an intestine includes at least one of regulating an intestinal function and regulating a gut microbiota.

In some embodiments, the regulating a gut microbiota includes regulating at least one of species, richness, evenness, and diversity of the gut microbiota.

In some embodiments, the regulating a gut microbiota includes regulating a change in at least one of the species, the richness, the evenness, and the diversity of the gut microbiota that is induced by a disease and/or a drug.

In some embodiments, the change in at least one of the species, the richness, the evenness, and the diversity of the gut microbiota that is induced by the disease and/or the drug includes a change in at least one of the species, the richness, the evenness, and the diversity of the gut microbiota that is induced by any one of the following D1) to D2): D1) an infectious disease, a tumor or cancer, an inflammation, an allergic disease, or an autoimmune disease; and D2) a drug for preventing and/or treating the D1).

In some embodiments, the disease in the D1) is an intestinal disease.

In some embodiments, the drug in the D2) is an anti-tumor drug.

In some embodiments, the anti-tumor drug in the D2) includes an immunotherapeutic drug.

In some embodiments, the immunotherapeutic drug in the D2) includes an immune checkpoint inhibitor.

In some embodiments, the immune checkpoint inhibitor in the D2) includes an inhibitor targeting at least one of PD-1, PD-L1, PD-L2, CTLA-4, TIM-3, LAG-3, TIGIT, VISTA, BTLA, CD27, CD28, CD70, CD80, CD86, CD137, CD276, KIRs, TNFRSF4, GITR, GITRL, 4-1BBL, A2aR, VTCN1, IDO, and KLRA.

In some embodiments, the disease mediated by the STING and/or NOD2 pathway includes at least one of an infectious disease, a tumor or cancer, an inflammation, an allergic disease, and an autoimmune disease.

In some embodiments, the pharmaceutical composition is administered at least once daily. In some embodiments, when the pharmaceutical composition is administered twice or more daily, the pharmaceutical composition is administered at a same dose. In some embodiments, when the pharmaceutical composition is administered twice or more daily, the pharmaceutical composition is administered at different doses. In some embodiments, a route for the administering includes at least one of oral administration, sublingual administration, nasal administration, rectal administration, inhalation administration, transdermal administration, intragastric administration, intraperitoneal injection, subcutaneous injection, and intramuscular injection. In some embodiments, when the pharmaceutical composition includes a microorganism-derived component and a drug for combination therapy, the microorganism-derived component and the drug for combination therapy can be administered simultaneously, separately, or sequentially.

The term "subject" or "patient" refers to any mammal. A subject or patient described as "in need" refers to a mammal requiring a treatment (or a prevention) of a disease. For example, the subject or patient in need includes any one or more animals under the class *Mammalia,* such as animals from *Monotremata, Marsupialia, Insectivora, Macroscelidea, Scandentia, Dermoptera, Chiroptera, Primates, Xenarthra, Pholidota, Lagomorpha, Rodentia, Carnivora, Sirenia, Hyracoidea, Tubulidentata, Perissodactyla, Artiodactyla, Cetacea,* etc. Common mammals include *Rodentia* (such as mice, rats, hamsters, and guinea pigs), *Lagomorpha* (such as rabbits), *Perissodactyla* (such as horses and donkeys), *Artiodactyla* (such as sheep, goats, camels, cattle, and pigs), *Primates* (such as monkeys, gorillas, chimpanzees, and humans), *Carnivora* (such as dogs and cats), etc. It can be understood that the subject may be healthy or may have a disease at any development stage.

The present disclosure further relates to a combined use of C1 and C2, including a use of the C1 and the C2 in preparation of an anti-tumor drug or for preventing or treating a tumor. The C1 includes at least one of a bacterial strain of a genus *Enterococcus,* a metabolite of the bacterial strain of the genus *Enterococcus,* a culture of the bacterial strain of the genus *Enterococcus,* and a fermentation supernatant of the bacterial strain of the genus *Enterococcus.* The C2 includes an immune checkpoint inhibitor. An anti-tumor activity of the anti-tumor drug includes prevention or treatment of at least one tumor of kidney cancer, liver cancer, and fibrosarcoma.

The term "treating" a disease in a subject or "treating" a subject who has or is suspected of having a disease refers to administering a medication to the subject, for example, administering one or more drugs to reduce or prevent the worsening of at least one symptom of the disease. Therefore, in an embodiment, the "treating" specifically refers to delaying progression, accelerating remission, inducing remission, increasing remission, accelerating recovery, enhancing efficacy of an alternative therapy, or reducing resistance to an alternative therapy, or a combination thereof for a tumor patient.

In some embodiments, the bacterial strain of the genus *Enterococcus* may be any of the aforementioned bacterial strains, for example, and the immune checkpoint inhibitor may also be any of the aforementioned immune checkpoint inhibitors.

In some embodiments, the bacterial strain of the genus *Enterococcus* is a live bacterium, an attenuated bacterium, a killed bacterium, a lyophilized bacterium, or an irradiated bacterium.

In some embodiments, the bacterial strain of the genus *Enterococcus* is lyophilized. In some embodiments, the bacterial strain of the genus *Enterococcus* is spray-dried. In some embodiments, the bacterial strain of the genus *Enterococcus* is lyophilized or spray-dried and is viable. In some embodiments, the bacterial strain of the genus *Enterococcus* is reconstituted prior to administration. In some cases, the reconstitution is conducted with a diluent as described herein.

The term "membrane vesicle" or "MV" refers to a composition derived from a bacterium. The MV includes bacterial lipids and bacterial proteins and/or bacterial nucleic acids and/or carbohydrate components encapsulated within a nanoparticle. The MV may include 1, 2, 3, 4, 5, 10, or more than 10 distinct lipid species. The MV may include 1, 2, 3, 4, 5, 10, or more than 10 distinct protein species. The MV may include 1, 2, 3, 4, 5, 10, or more than 10 distinct nucleic acid species.

Those skilled in the art can prepare MV from a bacterial culture using a method known to those skilled in the art (S. Bin Park et al., PLoS ONE 6(3): e17629 (2011)).

The embodiments of the present disclosure also relate to a method for preventing or treating at least one tumor of kidney cancer, liver cancer, and fibrosarcoma, including administering an effective dose of C1 and C2 to a subject. In some embodiments, the C1 and the C2 are administered at least once daily. In some embodiments, the C1 and the C2 are administered simultaneously, separately, or sequentially. In some embodiments, when the C1 or the C2 is administered twice or more daily, the C1 or the C2 is administered at a same dose. In some embodiments, when the C1 or the C2 is administered twice or more daily, the C1 or the C2 is administered at different doses. In some embodiments, a route for the administering includes at least one of oral administration, sublingual administration, nasal administration, rectal administration, inhalation administration, transdermal administration, intragastric administration, intraperitoneal injection, subcutaneous injection, and intramuscular injection.

The bacterial strain involved in the following examples is *Enterococcus lactis* MNC-168, which was deposited in the Guangdong Microbial Culture Collection Center (GDMCC) under an accession number GDMCC No. 61121 on August 05, 2020. The Guangdong Microbial Culture Collection Center (GDMCC) is located at the Guangdong Institute of Microbiology, Guangdong Academy of Sciences on No. 59 Building, No. 100, Xianliezhong Road, Guangzhou. A source, a screening method, physiological and biochemical characteristics, and a 16S rRNA sequence (SEQ ID NO: 1) of this strain are documented in WO2022156119A1 and are incorporated into the present disclosure by reference.

The above embodiments are illustrated below with reference to specific examples.

### Example 1: Preparation of MVs of Enterococcus lactis

### 1. Preparation of an MNC-168 supernatant

A glycerol stock of *Enterococcus lactis* MNC-168 was thawed at 37°C, and then inoculated onto an anaerobic blood agar plate within an anaerobic workstation for activation. An activated strain was inoculated into an MM01 liquid medium and cultured anaerobically to produce a sufficient number of viable bacteria. A resulting bacterial solution was centrifuged at 10,000 g for 10 min to make bacterial cells precipitated. A resulting supernatant was collected and filtered through a 0.22 µm filter membrane to obtain the MNC-168 supernatant, which was either used directly for subsequent experiments or frozen at -80°C.

### 2. Isolation of MVs

MVs were isolated from the MNC-168 supernatant using a bacterial MV isolation kit (Runji Biotechnology, #BacMV10-10).

### 3. Identification of MVs

(1) An isolated bacterial MV sample was diluted 200-fold with PBS, and a size and distribution of MVs were detected using Nanosight NS300.
(2) 5 µL of the isolated bacterial MV sample was taken and added dropwise to a copper mesh, incubated for 1 min, stained with 1% phosphotungstic acid for 2 min, rapidly rinsed twice each with one drop of pure water, dried at room temperature for 10 min, and then observed using TEM (at a voltage of 80 kV).
(3) The isolated bacterial MV sample was mixed with a DNA loading buffer, and a reaction was conducted at 95°C for 5 min. Then, 1% agarose gel electrophoresis was conducted to determine a size of DNA.

Results are shown in A to C of FIG. 1. The isolated MVs are round and have a diameter typically of about 100 nm. According to electrophoresis results, the MVs carry substantial DNA derived from bacteria, and have molecular weights predominantly concentrated at 100 bp or less.

### Example 2: Activation of STING pathway-dependent anti-tumor immunity by Enterococcus lactis MVs

STING reporter cell assay: THP-1 cells carrying an IFN-β promoter reporter (a cell line established by MoonBiotech) were used for evaluation. Specific steps were as follows:

THP-1-IFNβ-promoter reporter cells were inoculated into a 96-well plate at 1 × 10⁵ cells/well. A control group, an MNC-168 MV group, and an MNC-168 MV + H-151 group were set. The control group was a blank control. In the MNC-168 MV group, 5 µL of MVs (concentration: 4.5 × 10⁵/µL) was added. In the MNC-168 MV + H-151 group, 5 µL of MVs (concentration: 4.5 × 10⁵/µL) was added, and H-151 was added at a final concentration of 10 µM. Cells were then cultured for 24 h. Centrifugation was conducted at 300 g for 5 min. A resulting culture supernatant was discarded, 50 µL of a 1 × luciferase detection reagent was added, and a reaction was conducted for 1 min. A luminescence value was measured using a microplate reader. Relative luminescence values were normalized to the control group to evaluate the influence of MVs of *Enterococcus lactis* MNC-168 on the transcriptional activity of IFN-β.

RT-PCR assay: THP-1 cells were treated according to the experimental steps for the STING reporter cells. Cells undergoing different treatments were collected and subjected to total RNA extraction, and then reverse transcription and qPCR were conducted to determine expression levels of target genes.

Results are shown in D to F of FIG. 1. According to these results, the MVs of *Enterococcus lactis* MNC-168 can significantly enhance the STING activity 3-fold, and this activation can be suppressed by the STING-specific inhibitor H-151. Further, according to detection results for STING downstream target genes IFN-β and TNF-α, the MVs of *Enterococcus lactis* MNC-168 can also significantly activate the expression of the two target genes, with 5-fold and 4-fold increases, respectively. This activation can be similarly inhibited by the STING-specific inhibitor H-151.

According to these results, *Enterococcus lactis* MNC-168 can induce the STING pathway-dependent activation through MVs to up-regulate the expression of IFN-β and TNF-α that can suppress the proliferation, metastasis, and angiogenesis of tumor cells, promote the apoptosis of tumor cells, and activate the anti-tumor immune response, ultimately enhancing the anti-tumor immune activity.

### Example 3: Combination therapy of kidney cancer with MNC-168 and an anti-PD-1 antibody

The therapeutic efficacy of the combination therapy with the *Enterococcus lactis* MNC-168 and the anti-PD-1 antibody was validated in a mouse syngeneic Renca (kidney cancer) model. This experimental protocol had been reviewed and approved by the Institutional Animal Care and Use Committee of MoonBiotech.

Test bacterial strain: A glycerol stock of *Enterococcus lactis* MNC-168 was thawed at 37°C, and then inoculated onto an anaerobic blood agar plate in an anaerobic workstation for activation. An activated strain was inoculated into an MM01 liquid medium and cultured anaerobically to produce a sufficient number of viable bacteria. A resulting bacterial solution was concentrated through centrifugation, and then resuspended in a vehicle to produce a test material with a purity and viable count (1 × 10¹⁰ CFU/mL) required by an animal experiment.

Tumor cells: Renca mouse kidney cancer cells from Nanjing Kebai Biotechnology Co., Ltd.

Anti-PD-1 antibody: An anti-mPD-1 antibody (RMP1-14) from BioXcell was prepared with normal saline into a 2 mg/mL solution.

Experimental animals: 55 BALB/c mice aged 5 weeks from GemPharmatech Co., Ltd. (Nanjing).

Animal experiment: According to body weights of 20.0 g to 22.0 g, the BALB/c mice were divided into a control group and an *Enterococcus lactis* MNC-168 group with 27 to 28 mice in each group. On the day (day 0) of tumor inoculation, mice were intragastrically administered once daily with either a medium or a live *Enterococcus lactis* MNC-168 suspension at 2 × 10⁹ CFU/mouse. On the day (day 0) of tumor inoculation, mice were subcutaneously inoculated with Renca mouse kidney cancer cells at 5 × 10⁴ cells/mouse. When a mean tumor volume reached approximately 20 mm³ to 50 mm³ on day 5 after tumor inoculation, based on whether the anti-mPD-1 antibody (PD-1) was intraperitoneally injected, the mice were further divided into a control group, an anti-PD-1 antibody group, an MNC-168 group, and an MNC-168 + anti-PD-1 antibody group with 13 to 14 mice in each group. In the control group, the medium and a 0.9% sodium chloride injection were administered. In the anti-PD-1 antibody group, the medium and the anti-mPD-1 antibody were administered. In the MNC-168 group, the MNC-168 and the 0.9% sodium chloride injection were administered. In the MNC-168 + anti-PD-1 antibody group, the MNC-168 and the anti-mPD-1 antibody were administered. The medium or the live bacterial suspension was intragastrically administered once daily at 2 × 10⁹ CFU/0.2 mL/mouse. The 0.9% sodium chloride injection or the anti-mPD-1 antibody (PD-1) was intraperitoneally injected at 200 µg/0.1 mL/mouse on day 5, day 8, day 11, and day 14. During the animal experiment, an animal condition, a tumor change, a tumor size, and a mouse body weight were monitored regularly at specified time points. At an experimental endpoint, all surviving mice were dissected for sample collection. A tumor weight was measured, and a tumor volume was calculated. Statistical analysis and comparisons were performed, including a tumor volume change curve, a tumor volume at endpoint, a tumor weight at endpoint, and a TGI rate at endpoint. TGI rate = 1 - (individual tumor volume - individual tumor volume at grouping)/(mean tumor volume of control group - mean tumor volume of control group at grouping) × 100%. Data was expressed as mean ± SD. Plotting and statistical analysis were performed using GraphPad Prism 8.0.2 software. For comparisons among three or more groups, statistical analysis was performed using one-way ANOVA in combination with Tukey's test for multiple comparisons. For two-factor evaluation, statistical analysis was performed using two-way ANOVA (2way ANOVA) in combination with Sidak's test for multiple comparisons. A statistical significance was indicated by *: *p < 0.05, **p < 0.01, ***p < 0.001, and ****p < 0.0001.

Test results are shown in the following table:

**Table 1 Experimental results of the combination therapy of kidney cancer with the MNC-168 and anti-PD-1 antibody**

| Group | Volume (mm³) | Weight (g) | TGI rate (%) | Response rate (%) | Eradication rate (%) |
|---|---|---|---|---|---|
| Control | 2193 | 2.188 | 0 | 20.0 | 0 |
| PD-1 | 1195 | 1.102 | 45.50 | 50.0 | 0 |
| MNC-168 | 883 | 0.8880 | 59.57 | 85.7 | 14.3 |
| MNC-168 + PD-1 | 398 | 0.3786 | 81.88 | 87.5 | 37.5 |

Results are shown in Table 1 and FIG. 2 to FIG. 6. According to the results, during the animal experiment, a tumor volume of the MNC-168 + anti-PD-1 antibody group is significantly smaller than a tumor volume of the anti-PD-1 antibody group (as shown in FIG. 2). At the experimental endpoint, mice in the control group have a mean tumor volume of 2,193 mm³ and a mean tumor weight of 2.188 g, mice in the anti-PD-1 antibody group have a mean tumor volume of 1,195 mm³ and a mean tumor weight of 1.102 g, mice in the MNC-168 group have a mean tumor volume of 883 mm³ and a mean tumor weight of 0.8880 g, and mice in the MNC-168 + anti-PD-1 antibody group have a mean tumor volume of 398 mm³ and a mean tumor weight of 0.3786 g. At the experimental endpoint, a tumor volume and a tumor weight of mice are significantly reduced in the MNC-168 + anti-PD-1 antibody group compared to the anti-PD-1 antibody group (FIG. 3 and FIG. 4). At the experimental endpoint, a TGI rate of the anti-PD-1 antibody group is 45.50%, and a TGI rate of the MNC-168 + anti-PD-1 antibody group is 81.88% that is significantly higher than the TGI rate of the anti-PD-1 antibody group (FIG. 5). At the experimental endpoint, a response rate of the anti-PD-1 antibody group is 50.0% and a response rate of the MNC-168 + anti-PD-1 antibody group is 87.5%. It can be seen that the combined administration of the MNC-168 and anti-PD-1 antibody significantly enhances the anti-tumor response rate of the anti-PD-1 antibody (FIG. 6). Additionally, a tumor cure rate (eradication rate) is 0% in the anti-PD-1 antibody group and 37.5% in the MNC-168 + anti-PD-1 antibody group. It can be seen that the combined administration of the MNC-168 and anti-PD-1 antibody significantly improves the tumor cure rate of the anti-PD-1 antibody. Therefore, these results reveal that MNC-168 can significantly enhance the therapeutic efficacy of the anti-PD-1 antibody against kidney cancer.

### Example 4: Combination therapy of fibrosarcoma with MNC-168 and an anti-PD-1 antibody

The therapeutic efficacy of the combination therapy with the MNC-168 and the anti-PD-1 antibody was validated in a mouse syngeneic WEHI 164 (fibrosarcoma) model. This experimental protocol had been reviewed and approved by the Institutional Animal Care and Use Committee of MoonBiotech.

Test bacterial strain: A glycerol stock of *Enterococcus lactis* MNC-168 was thawed at 37°C, and then inoculated onto an anaerobic blood agar plate in an anaerobic workstation for activation. An activated strain was inoculated into an MM01 liquid medium and cultured anaerobically to produce a sufficient number of viable bacteria. A resulting bacterial solution was concentrated through centrifugation, and then resuspended in a vehicle to produce a test material with a purity and viable count (1 × 10¹⁰ CFU/mL) required by an animal experiment.

Tumor cells: WEHI 164 mouse fibrosarcoma cells from Nanjing Kebai Biotechnology Co., Ltd.

Anti-PD-1 antibody: An anti-mPD-1 antibody (RMP1-14) from BioXcell was prepared with normal saline into a 2 mg/mL solution.

Experimental animals: 55 BALB/c mice aged 5 weeks from GemPharmatech Co., Ltd. (Nanjing).

Animal experiment: According to body weights of 20.0 g to 22.0 g, the BALB/c mice were divided into a control group and an *Enterococcus lactis* MNC-168 group with 27 to 28 mice in each group. On the day (day 0) of tumor inoculation, mice were intragastrically administered once daily with either a medium or a live *Enterococcus lactis* MNC-168 suspension at 2 × 10⁹ CFU/mouse. On the day (day 0) of tumor inoculation, mice were subcutaneously inoculated with WEHI 164 mouse fibrosarcoma cells at 5 × 10⁴ cells/mouse. When a mean tumor volume reached approximately 40 mm³ to 100 mm³ on day 5 after tumor inoculation, based on whether the anti-mPD-1 antibody (PD-1) was intraperitoneally injected, the mice were further divided into a control group, an anti-PD-1 antibody group, an MNC-168 group, and an MNC-168 + anti-PD-1 antibody group with 13 to 14 mice in each group. In the control group, the medium and a 0.9% sodium chloride injection were administered. In the anti-PD-1 antibody group, the medium and the anti-mPD-1 antibody were administered. In the MNC-168 group, the MNC-168 and the 0.9% sodium chloride injection were administered. In the MNC-168 + anti-PD-1 antibody group, the MNC-168 and the anti-mPD-1 antibody were administered. The medium or the live bacterial suspension was intragastrically administered once daily at 2 × 10⁹ CFU/0.2 mL/mouse. The 0.9% sodium chloride injection or the anti-mPD-1 antibody (PD-1) was intraperitoneally injected at 200 µg/0.1 mL/mouse on day 5, day 8, day 11, and day 14. During the animal experiment, an animal condition, a tumor change, a tumor size, and a mouse body weight were monitored regularly at specified time points. At an experimental endpoint, all surviving mice were dissected for sample collection. A tumor weight was measured, and a tumor volume was calculated. Statistical analysis and comparisons were performed, including a tumor volume change curve, a tumor volume at endpoint, and a TGI rate at endpoint. TGI rate = 1 - (individual tumor volume - individual tumor volume at grouping)/(mean tumor volume of control group - mean tumor volume of control group at grouping) × 100%. Data was expressed as mean ± SD. Plotting and statistical analysis were performed using GraphPad Prism 8.0.2 software. For comparisons among three or more groups, statistical analysis was performed using one-way ANOVA in combination with Tukey's test for multiple comparisons. For two-factor evaluation, statistical analysis was performed using two-way ANOVA (2way ANOVA) in combination with Sidak's test for multiple comparisons. A statistical significance was indicated by *: *p < 0.05, **p < 0.01, ***p < 0.001, and ****p < 0.0001.

Test results are shown in the following table:

**Table 2 Experimental results of the combination therapy of fibrosarcoma with the MNC-168 and anti-PD-1 antibody**

| Group | Volume (mm³) | TGI rate (%) | Response rate (%) | Eradication rate (%) |
|---|---|---|---|---|
| Control | 2320 | 0 | 00.0 | 0 |
| PD-1 | 2061 | 11 | 40.0 | 0 |
| MNC-168 | 1334 | 42 | 50.0 | 12.5 |
| MNC-168 + PD-1 | 771 | 67 | 57.1 | 14.3 |

Results are shown in Table 2 and FIG. 7 to FIG. 10. According to the results, during the animal experiment, a tumor volume of the MNC-168 + anti-PD-1 antibody group is significantly smaller than a tumor volume of the anti-PD-1 antibody group (as shown in FIG. 7). At the experimental endpoint, mice in the control group have a mean tumor volume of 2,320 mm³, mice in the anti-PD-1 antibody group have a mean tumor volume of 2,061 mm³, and mice in the MNC-168 + anti-PD-1 antibody group have a mean tumor volume of 771 mm³. At the experimental endpoint, a tumor volume of mice is significantly reduced in the MNC-168 + anti-PD-1 antibody group compared to the anti-PD-1 antibody group (FIG. 8). At the experimental endpoint, a TGI rate of the anti-PD-1 antibody group is 11%, and a TGI rate of the MNC-168 + anti-PD-1 antibody group is 67% that is significantly higher than the TGI rate of the anti-PD-1 antibody group (FIG. 9). At the experimental endpoint, a response rate of the anti-PD-1 antibody group is 40% and a response rate of the MNC-168 + anti-PD-1 antibody group is 57%. It can be seen that the combined administration of the MNC-168 and anti-PD-1 antibody significantly enhances the anti-tumor response rate of the anti-PD-1 antibody (FIG. 10). Additionally, a tumor cure rate (eradication rate) is 0% in the anti-PD-1 antibody group and 14.3% in the MNC-168 + anti-PD-1 antibody group. It can be seen that the combined administration of the MNC-168 and anti-PD-1 antibody significantly improves the tumor cure rate of the anti-PD-1 antibody. Therefore, these results reveal that MNC-168 can significantly enhance the therapeutic efficacy of the anti-PD-1 antibody against fibrosarcoma.

### Example 5: Combination therapy of liver cancer with MNC-168 and an anti-PD-1 antibody

The therapeutic efficacy of the combination therapy with the MNC-168 and the anti-PD-1 antibody was validated in a mouse syngeneic tumor H22 (liver cancer) model. This experimental protocol had been reviewed and approved by the Institutional Animal Care and Use Committee of MoonBiotech.

Test bacterial strain: A glycerol stock of *Enterococcus lactis* MNC-168 was thawed at 37°C, and then inoculated onto an anaerobic blood agar plate in an anaerobic workstation for activation. An activated strain was inoculated into an MM01 liquid medium and cultured anaerobically to produce a sufficient number of viable bacteria. A resulting bacterial solution was concentrated through centrifugation, and then resuspended in a vehicle to produce a test material with a purity and viable count (1 × 10¹⁰ CFU/mL) required by an animal experiment.

Tumor cells: H22 mouse liver cancer cells from Nanjing Kebai Biotechnology Co., Ltd.

Anti-PD-1 antibody: An anti-mPD-1 antibody (RMP1-14) from BioXcell was prepared with normal saline into a 2 mg/mL solution.

### Experimental animals: 45 C57BL/6J mice aged 4 weeks from GemPharmatech Co., Ltd. (Nanjing).

Animal experiment: According to body weights of 20.0 g to 22.0 g, the C57BL/6J mice were divided into a control group, an anti-PD-1 antibody group, and an MNC-168 + anti-PD-1 antibody group. On the day (day 0) of tumor inoculation, mice were intragastrically administered once daily with either a medium or a live *Enterococcus lactis* MNC-168 suspension at 2 × 10⁹ CFU/mouse. On the day (day 0) of tumor inoculation, mice were subcutaneously inoculated with H22 mouse liver cancer cells at 5 × 10⁵ cells/mouse. When a mean tumor volume reached approximately 50 mm³ on day 6 after tumor inoculation, the medium and a 0.9% sodium chloride injection were administered in the control group, the medium and the anti-mPD-1 antibody were administered in the anti-PD-1 antibody group, and the MNC-168 and the anti-mPD-1 antibody were administered in the MNC-168 + anti-PD-1 antibody group. The medium or the live bacterial suspension was intragastrically administered once daily at 2 × 10⁹ CFU/0.2 mL/mouse. The 0.9% sodium chloride injection or the anti-mPD-1 antibody (PD-1) was intraperitoneally injected at 200 µg/0.1 mL/mouse on day 6, day 9, day 12, and day 16. During the animal experiment, an animal condition, a tumor change, a tumor size, and a mouse body weight were monitored regularly at specified time points. At an experimental endpoint, all surviving mice were dissected for sample collection. A tumor weight was measured, and a tumor volume was calculated. Statistical analysis and comparisons were performed, including a tumor volume change curve, a tumor volume at endpoint, a tumor weight at endpoint, and a TGI rate at endpoint. TGI rate = 1 - (individual tumor volume - individual tumor volume at grouping)/(mean tumor volume of A group - mean tumor volume of A group at grouping) × 100%. Data was expressed as mean ± SD. Plotting and statistical analysis were performed using GraphPad Prism 8.0.2 software. For comparisons among three or more groups, statistical analysis was performed using one-way ANOVA in combination with Tukey's test for multiple comparisons. For two-factor evaluation, statistical analysis was performed using two-way ANOVA (2way ANOVA) in combination with Sidak's test for multiple comparisons. A statistical significance was indicated by *: *p < 0.05, **p < 0.01, ***p < 0.001, and ****p < 0.0001.

**Table 3 Experimental results of the combination therapy of liver cancer with the MNC-168 and anti-PD-1 antibody**

| Group | Volume (mm³) | Weight (g) | TGI rate (%) | Response rate (%) |
|---|---|---|---|---|
| Control | 1858 | 2.065 | -14.55 | 0 |
| PD-1 | 1508 | 1.548 | 18.75 | 0 |
| MNC-168 | 1553 | 1.503 | 16.67 | 16.7 |
| MNC-168 + PD-1 | 748 | 0.5367 | 59.71 | 100 |

Results are shown in Table 3 and FIG. 11 to FIG. 15. According to the results, during the animal experiment, a tumor volume of the MNC-168 + anti-PD-1 antibody group is significantly smaller than a tumor volume of the anti-PD-1 antibody group (as shown in FIG. 11). At the experimental endpoint, mice in the control group have a mean tumor volume of 1,858 mm³ and a mean tumor weight of 2.065 g, mice in the anti-PD-1 antibody group have a mean tumor volume of 1,508 mm³ and a mean tumor weight of 1.548 g, and mice in the MNC-168 + anti-PD-1 antibody group have a mean tumor volume of 748 mm³ and a mean tumor weight of 0.5367 g. At the experimental endpoint, a tumor volume and a tumor weight of mice are significantly reduced in the MNC-168 + anti-PD-1 antibody group compared to the anti-PD-1 antibody group (FIG. 12 and FIG. 13). At the experimental endpoint, a TGI rate of the anti-PD-1 antibody group is 18.75%, and a TGI rate of the MNC-168 + anti-PD-1 antibody group is 59.71% that is significantly higher than the TGI rate of the anti-PD-1 antibody group (FIG. 14). At the experimental endpoint, a response rate of the anti-PD-1 antibody group is 0% and a response rate of the MNC-168 + anti-PD-1 antibody group is 100%. It can be seen that the combined administration of the MNC-168 and anti-PD-1 antibody significantly enhances the anti-tumor response rate (FIG. 15). Therefore, these results reveal that MNC-168 can significantly enhance the therapeutic efficacy of the anti-PD-1 antibody against liver cancer.

### Example 6: Promotion of STING pathway-dependent anti-tumor immunity by a culture supernatant of Enterococcus lactis

Preparation of an MNC-168 supernatant: A glycerol stock of *Enterococcus lactis* MNC-168 was thawed at 37°C, and then inoculated onto an anaerobic blood agar plate within an anaerobic workstation for activation. An activated strain was inoculated into an MM01 liquid medium and cultured anaerobically to produce a sufficient number of viable bacteria. A resulting bacterial solution was centrifuged at 10,000 g for 10 min to make bacterial cells precipitated. A resulting supernatant was collected and filtered through a 0.22 µm filter membrane to obtain the MNC-168 supernatant, which was either used directly for subsequent experiments or frozen at - 80°C.

STING reporter cell assay: THP-1 cells carrying an IFN-β promoter reporter (a cell line established by MoonBiotech) were used for evaluation. Specific steps were as follows:

THP-1-IFNβ-promoter reporter cells were inoculated into a 96-well plate at 1 × 10⁵ cells/well. A control group, an MNC-168 SP group, and an MNC-168 SP + H-151 group were set. In the control group, 10 µL of an MM01 medium was added. In the MNC-168 SP group, 10 µL of the MNC-168 supernatant was added. In the MNC-168 SP + H-151 group, 10 µL of an MNC-168 supernatant including H-151 at a final concentration of 10 µM was added. Cells were then cultured for 24 h. Centrifugation was conducted at 300 g for 5 min. A resulting culture supernatant was discarded, 50 µL of a 1 × luciferase detection reagent was added, and a reaction was conducted for 1 min. A luminescence value was measured using a microplate reader. Relative luminescence values were normalized to the control group to evaluate the influence of the MNC-168 supernatant of *Enterococcus lactis* on the transcriptional activity of IFN-β.

RT-PCR assay: THP-1 cells were treated according to the experimental steps for the STING reporter cells. Cells undergoing different treatments were collected and subjected to total RNA extraction, and then reverse transcription and qPCR were conducted to determine expression levels of target genes.

Results are shown in A to C of FIG. 16. According to these results, the supernatant of *Enterococcus lactis* MNC-168 can significantly enhance the STING activity 2-fold, and this activation can be suppressed by the STING-specific inhibitor H-151. Further, according to detection results for STING downstream target genes IFN-β and TNF-α, the supernatant of *Enterococcus lactis* MNC-168 can also significantly activate the expression of the two target genes, with approximately a 4-fold increase. This activation can be similarly inhibited by the STING-specific inhibitor H-151.

According to these results, the supernatant of *Enterococcus lactis* MNC-168 can induce the STING pathway-dependent activation to up-regulate the expression of IFN-β and TNF-α that can suppress the proliferation and metastasis of tumor cells, promote the apoptosis of tumor cells, and activate the anti-tumor immune response, ultimately enhancing the anti-tumor immune activity.

### Example 7: Promotion of NOD2 pathway-dependent anti-tumor immunity by a culture supernatant of Enterococcus lactis

Preparation of an MNC-168 supernatant: The MNC-168 supernatant prepared in Example 6 was adopted.

NOD2 activation evaluation experiment: THP-1 cells were inoculated into a 12-well plate at 5 × 10⁵ cells/well. A control group, an MNC-168 SP group, and an MNC-168 SP + GSK717 group were set. In the control group, 50 µL of an MM01 medium was added. In the MNC-168 SP group, 50 µL of the MNC-168 supernatant was added. In the MNC-168 SP + GSK717 group, 50 µL of an MNC-168 supernatant including GSK717 at a final concentration of 10 µM was added. Cells were then cultured for 24 h. Centrifugation was conducted at 300 g for 5 min. A resulting culture supernatant was discarded, 50 µL of a 1 × luciferase detection reagent was added, and a reaction was conducted for 1 min. A luminescence value was measured using a microplate reader. Relative luminescence values were normalized to the control group to evaluate the influence of the MNC-168 supernatant of *Enterococcus lactis* on the activity of NOD2.

RT-PCR assay: With reference to the RT-PCR experimental steps in Example 6, total RNA extraction, reverse transcription, and qPCR were conducted to determine expression levels of target genes.

Results are shown in A and B of FIG. 17. According to these results, the supernatant of *Enterococcus lactis* MNC-168 can significantly up-regulate the expression of NOD2 downstream target genes IL-1β and IL-6, with more than 20-fold and 10-fold increases, respectively. This activation can also be partially inhibited by the NOD2-specific inhibitor GSK717.

According to these results, the supernatant of *Enterococcus lactis* MNC-168 can partly induce the NOD2 pathway-dependent activation to up-regulate the expression of IFN-β and TNF-α that can suppress the proliferation and metastasis of tumor cells, promote the apoptosis of tumor cells, and activate the anti-tumor immune response, ultimately enhancing the anti-tumor immune activity.

### Example 8: Promotion of the activation of systemic immunity by Enterococcus lactis

The therapeutic efficacy of the combination therapy with *Enterococcus lactis* MNC-168 and an anti-PD-1 antibody and the influence of the combination therapy on cytokines and chemokines in mouse serum were validated in a mouse syngeneic tumor MC38 (colorectal cancer) model. This experimental protocol had been reviewed and approved by the Institutional Animal Care and Use Committee of MoonBiotech.

Test bacterial strain: A glycerol stock of *Enterococcus lactis* MNC-168 was thawed at 37°C, and then inoculated onto an anaerobic blood agar plate in an anaerobic workstation for activation. An activated strain was inoculated into an MM01 liquid medium and cultured anaerobically to produce a sufficient number of viable bacteria. A resulting bacterial solution was concentrated through centrifugation, and then resuspended in a vehicle to produce a test material with a purity and viable count (1 × 10¹⁰ CFU/mL) required by an animal experiment.

Tumor cells: MC38 mouse colorectal cancer cells from Nanjing Kebai Biotechnology Co., Ltd.

Anti-PD-1 antibody: An anti-mPD-1 antibody (RMP1-14) from BioXcell was prepared with normal saline into a 2 mg/mL solution.

Experimental animals: 55 C57BL/6J mice aged 5 weeks from GemPharmatech Co., Ltd. (Nanjing).

Animal experiment: According to body weights of 20.0 g to 22.0 g, the C57BL/6J mice were divided into a control group and an *Enterococcus lactis* MNC-168 group with 27 to 28 mice in each group. On the day (day 0) of tumor inoculation, mice were intragastrically administered once daily with either a medium or a live *Enterococcus lactis* MNC-168 suspension at 2 × 10⁹ CFU/mouse. On the day (day 0) of tumor inoculation, mice were subcutaneously inoculated with Renca mouse kidney cancer cells at 5 × 10⁴ cells/mouse. When a mean tumor volume reached approximately 20 mm³ to 50 mm³ on day 5 after tumor inoculation, based on whether the anti-mPD-1 antibody (PD-1) was intraperitoneally injected, the mice were further divided into a control group, an anti-PD-1 antibody group, an MNC-168 group, and an MNC-168 + anti-PD-1 antibody group with 13 to 14 mice in each group. In the control group, the medium and a 0.9% sodium chloride injection were administered. In the anti-PD-1 antibody group, the medium and the anti-mPD-1 antibody were administered. In the MNC-168 group, the MNC-168 and the 0.9% sodium chloride injection were administered. In the MNC-168 + anti-PD-1 antibody group, the MNC-168 and the anti-mPD-1 antibody were administered. The medium or the live bacterial suspension was intragastrically administered once daily at 2 × 10⁹ CFU/0.2 mL/mouse. The 0.9% sodium chloride injection or the anti-mPD-1 antibody (PD-1) was intraperitoneally injected at 200 µg/0.1 mL/mouse on day 5, day 8, day 11, and day 14. During the animal experiment, an animal condition, a tumor change, a tumor size, and a mouse body weight were monitored regularly at specified time points. At an experimental endpoint, blood was collected from the mice, and serum was isolated. Concentrations of the relevant cytokines and chemokines in the serum were detected using ELISA kits.

Results are shown in A of FIG. 18. It can be seen that MNC-168 can significantly enhance the therapeutic efficacy of the anti-PD-1 antibody against MC38. Changes of cytokines and chemokines in serum were further determined by ELISA. Results are shown in B to E of FIG. 18. It can be known that the combination therapy with the MNC-168 and anti-PD-1 antibody can further enhance the increases in serum concentrations of CXCL1, CXCL9, CXCL10, and IFNγ compared to the anti-PD-1 antibody monotherapy.

### Example 9: Maintenance of the gut microbiota diversity in tumor-bearing mice by Enterococcus lactis

Collection of mouse fecal samples: Fecal samples were collected from the mouse syngeneic tumor MC38 (colorectal cancer) model in Example 8 and stored at -80°C for subsequent experiments, including fecal samples from the control (CT) group, the anti-PD-1 antibody group, the MNC-168 group, and the MNC-168 + anti-PD-1 antibody group before (pre) and after (post) the tumor inoculation and treatment, namely, preCT, prePD-1, preMNC-168, preMNC-168 + PD-1, postCT, postPD-1, postMNC-168, and postMNC-168-PD-1.

16S RNA sequencing analysis: The mouse fecal samples were sent to a sequencing company for DNA extraction, 16S ribosomal RNA gene amplification, Illumina MiSeq sequencing, bacterial identification, and abundance analysis, and finally, the bacterial diversity was analyzed.

Results are shown in FIG. 19. Prior to the tumor inoculation and treatment, that is, among the groups (pre), there are no differences in the species and diversity of gut microbiota in mice. However, according to the comparison between the preCT and postCT of the CT group after the inoculation of MC-38 colorectal cancer cells, the species and diversity of gut microbiota in mice of the postCT group significantly decline. After the inoculation of MC-38 and then the administration of MNC-168, that is, in the postMNC-168, the species and diversity of gut microbiota in mice do not significantly change compared to the preMNC-168 before the inoculation of MC-38. Compared to the changes in gut microbiota before and after tumor inoculation in the CT group, the MNC-168 treatment can restore the balance of gut microbiota. Results of the administration of the anti-PD-1 antibody or the MNC-168 + anti-PD-1 antibody were similar to those of the administration of MNC-168 (A in FIG. 19). The PCA analysis also indicates the similar findings: In the CT group, the diversity of gut microbiota in mice changes after tumor cell inoculation. However, in the MNC-168 treatment group, the diversity levels of gut microbiota in mice before and after tumor cell inoculation remain similar (B in FIG. 19). It can be known that MNC-168 can restore the gut microbiota in mice.

According to these results, MNC-168 can enhance the therapeutic efficacy of the anti-PD-1 antibody, and the combined administration of the MNC-168 and anti-PD-1 antibody further promotes the enhancement of systemic immunity in an organism, thereby boosting the overall immunity of the organism. Consequently, this approach holds therapeutic potential for various tumors or cancers including soft tissue tumors, hematologic tumors, glandular tumors, and metastatic tumors. Moreover, MNC-168 can mitigate the reduction in gut microbiota diversity induced by the anti-PD-1 antibody, thereby effectively maintaining the balance of gut microbiota.

MVs, as secretions of the gut bacteria, include a large amount of gut bacterial DNA and substances such as specific proteins. These specific components can activate the immune system of the entire organism, thereby enabling the prevention and treatment of various tumors or cancers, including solid tumors, soft tissue tumors, hematologic tumors, glandular tumors, and metastatic tumors. These specific components demonstrate a prominent immunomodulatory or immunostimulatory effect and a gut regulatory effect, and even can achieve superior outcomes to the direct administration of the gut bacteria. Further, components of the bacterial strain, including a supernatant and an MV of the bacterial strain, can all activate the STING and/or NOD2 pathway. Therefore, when these components of the bacterial strain are prepared into drugs, diverse dosage forms can be adaptively provided based on different needs. Components derived from the bacterial strain, such as the bacterial strain, a metabolite of the bacterial strain, a supernatant of the bacterial strain, and MV secreted by the bacterial strain, can all exert prominent anti-tumor efficacy.

For clinically challenging tumors such as kidney cancer, fibrosarcoma, and liver cancer, achieving effective anti-tumor outcomes with drugs remains highly unpredictable. However, in the embodiments of the present disclosure, combinations of components of *Enterococcus* bacteria with immune checkpoint inhibitors can play a significant therapeutic role. Although these tumor lesions originate outside the gastrointestinal tract, *Enterococcus* bacteria originating from the gut, such as MNC-168, can still be used in combination with immune checkpoint inhibitors to enhance an anti-tumor effect, resulting in improved anti-tumor efficacy. It can be known that the combination of *Enterococcus* bacteria with immune checkpoint inhibitors such as an anti-PD-1 antibody significantly enhances the efficacy of the immune checkpoint inhibitors. Experimental data also reveals that the intervention with the bacterial strain of the present disclosure significantly increases the tumor eradication rate.

In the embodiments of the present disclosure, experiments for the supernatant and MV of the bacterial strain show that the supernatant and MV increase the STING activity 3-fold compared to a control. This may indicate that nucleic acid fragments (such as DNA fragments) present in the supernatant and MV specifically bind to C-Gas, thereby effectively activating the STING and/or NOD2 pathway to exert anti-tumor activity.

### Example 10: Treatment of colorectal cancer with MVs of Enterococcus lactis

The therapeutic efficacy of MNC-168 MVs was validated in a mouse syngeneic tumor CT-26 (colorectal cancer) model. This experimental protocol had been reviewed and approved by the Institutional Animal Care and Use Committee of MoonBiotech.

Test bacterial strain: A glycerol stock of *Enterococcus lactis* MNC-168 was thawed at 37°C, and then inoculated onto an anaerobic blood agar plate in an anaerobic workstation for activation. An activated strain was inoculated into an MM01 liquid medium and cultured anaerobically to produce a sufficient number of viable bacteria. A resulting bacterial solution was concentrated through centrifugation, and then resuspended in a vehicle to produce a test material with a purity and viable count (1 × 10¹⁰ CFU/mL) required by an animal experiment.

Preparation of MNC-168 MVs (MNC-168 MVs): The preparation method in Example 1 was adopted. The MVs were diluted with PBS to a required experimental concentration.

Tumor cells: CT-26 mouse colorectal cancer cells from the American Type Culture Collection (ATCC). The cells were inoculated at 1 × 10⁶ cells/mouse.

Experimental animals: BALB/c mice aged 8 weeks from Guangdong Vital River Laboratory Animal Technology Co., Ltd.

Administration route and dose: 50 µL of an MNC-168 MV solution including 10 µg of MVs was intratumorally injected once every two days for a total of four times. PBS was injected in a control group.

Animal experiment: A number of female BALB/c mice were ordered for this study. After a quarantine period, tumor cells were inoculated to establish a CT26 colorectal cancer ectopic syngeneic tumor model. The day of tumor inoculation was designated as day 0. Tumor measurement was started from day 5. When a mean tumor volume reached 100 mm³, mice with abnormal tumor growth were excluded, with a minimum of 12 mice with abnormal tumor growth. Qualified mice were then randomly divided according to tumor volumes. MNC-168 MVs were intratumorally injected on the day of grouping. During the animal experiment, an animal condition, a tumor change, a tumor size, and a mouse body weight were regularly monitored. At an experimental endpoint, tumor volume and weight changes were analyzed. Throughout the animal experiment, all mice had a body weight steadily increased. Data was expressed as mean ± SD. Plotting and statistical analysis were performed using GraphPad Prism 8.0.2 software. For comparisons among three or more groups, statistical analysis was performed using one-way ANOVA in combination with Tukey's test for multiple comparisons. For two-factor evaluation, statistical analysis was performed using two-way ANOVA (2way ANOVA) in combination with Sidak's test for multiple comparisons. A statistical significance was indicated by *: *p < 0.05, **p < 0.01, ***p < 0.001, and ****p < 0.0001.

**Table 4 Therapeutic outcomes of MNC-168 MV for CT-26 mouse colorectal cancer**

| Group | Volume (mm³) | Weight (g) |
|---|---|---|
| Control | 1053 | 1.043 |
| MNC-168 MV | 680.5 | 0.645 |

According to the experimental results shown in Table 4 and FIG. 20A, a tumor volume of the MNC-168 MV intratumoral injection group is significantly smaller than a tumor volume of the control group. At the experimental endpoint, a tumor volume in the control group is 1,053 mm³ and a tumor volume in the MNC-168 MV group is 680.5 mm³. Further, according to photographing and weighing results after dissection at the experimental endpoint, a tumor weight in the MNC-168 MV group is significantly smaller than a tumor weight in the control group (FIG. 20B), with a tumor weight of 1.043 g in the control group and a tumor weight of 0.645 g in the MNC-168 MV group. These experimental results show that MNC-168 MVs possess the ability to inhibit the growth of the CT-26 tumor, indicating that MNC-168 can play an anti-tumor role by secreting MVs.

### Example 11: Biomarker detection during the treatment of colorectal cancer with MVs of Enterococcus lactis

In this study, changes in immune cell populations in Example 10 were detected and analyzed by flow cytometry to elucidate an anti-tumor mechanism of the MVs of *Enterococcus lactis.*

Preparation of a tumor single-cell suspension:
1) Preparation of a tumor tissue homogenate: A tumor tissue was mechanically minced, homogenized, digested, and ground to produce a tumor homogenate.
2) The tumor homogenate was centrifuged in a centrifuge tube for 5 min at 500 g and room temperature. The centrifuge tube was then inverted on absorbent paper for 5 s to 10 s to remove a resulting supernatant.
2) 10 mL of a red blood cell lysis buffer was added, and red blood cell lysis was conducted for 10 min.
3) PBS was added to a 30 mL mark of the 50 mL centrifuge tube.
4) The centrifuge tube was centrifuged for 5 min at 500 g and room temperature and then inverted on absorbent paper to remove a resulting supernatant.
5) A resulting cell pellet was resuspended in PBS, and a resulting cell suspension was filtered through a filter membrane with a pore size of 70 µm to produce the tumor single-cell suspension for subsequent cell staining and flow cytometry analysis.

The cell staining and flow cytometry analysis were conducted with reference to Table 5.

**Table 5 Flow cytometry analysis indices**

| **Detection content** | **Detected target molecules** |
|---|---|
| DC cells | CD45, CD11c, MHCII |
| Secretion of IFN-γ/TNF-α by T cells | IFN-γ+/CD4+; IFNγ+/CD8+; TNF-α/CD4+; TNF-α/CD8+ |
| TAM cells | CD11b, F4/80, CD206, CD86 |

According to flow cytometry results, a quantity of DC cells significantly increases in the MNC-168 MV group compared to the control group (FIG. 21A). Further, according to TAM analysis, a ratio of CD206-expressing M2 TAMs to CD86-expressing M1 TAMs is significantly reduced in the MNC-168 MV group, indicating that MNC-168 MV suppresses M2 TAM polarization (FIG. 21B). It can also be seen from the analysis of cytokines in tumor-infiltrating T cells that the expression of IFN-γ by CD8⁺ T cells (cytotoxic T cells) is significantly up-regulated in the MNC-168 MV group (FIG. 21C). These results reveal that MNC-168 MVs can enhance the anti-tumor efficacy by promoting both innate immunity and adaptive immunity. Based on the previous experimental results, these results show that MNC-168 can enhance the anti-tumor efficacy by secreting MVs to promote the activation of both innate immunity and adaptive immunity *in vivo.*

The present disclosure has been described in detail above with reference to the examples, but the present disclosure is not limited to the above examples. Within the scope of knowledge possessed by those of ordinary skill in the art, various modifications can be made without departing from the purpose of the present disclosure. In addition, in the case of no conflict, the examples of the present disclosure and the features in the examples may be combined with each other.

## Claims

1. A composition, comprising a microorganism-derived component,
wherein preferably, the microorganism-derived component comprises a membrane vesicle (MV) isolated from a bacterial strain, and more preferably, the MV comprises at least one of a lipid, a protein, and a nucleic acid fragment;
preferably, the nucleic acid fragment comprises a DNA fragment;
preferably, the microorganism-derived component comprises at least one of following (1) to (5): (1) a bacterial strain with an immunostimulatory or immunomodulatory function, (2) a culture of the bacterial strain with the immunostimulatory or immunomodulatory function, (3) a fermentation supernatant of the bacterial strain with the immunostimulatory or immunomodulatory function, (4) a metabolite of the bacterial strain with the immunostimulatory or immunomodulatory function, and (5) a short-chain fatty acid from the bacterial strain with the immunostimulatory or immunomodulatory function;
preferably, the microorganism-derived component comprises the MV isolated from the bacterial strain and further comprises at least one of following (1) to (5): (1) the bacterial strain with the immunostimulatory or immunomodulatory function, (2) the culture of the bacterial strain with the immunostimulatory or immunomodulatory function, (3) the fermentation supernatant of the bacterial strain with the immunostimulatory or immunomodulatory function, (4) the metabolite of the bacterial strain with the immunostimulatory or immunomodulatory function, and (5) the short-chain fatty acid from the bacterial strain with the immunostimulatory or immunomodulatory function;
preferably, the microorganism-derived component comprises at least 50 wt%, 60 wt%, 70 wt%, 80 wt%, 85 wt%, 90 wt%, 95 wt%, 96 wt%, 97 wt%, 98 wt%, or 99 wt% and/or 50 v/v%, 60 v/v%, 70 v/v%, 80 v/v%, 85 v/v%, 90 v/v%, 95 v/v%, 96 v/v%, 97 v/v%, 98 v/v%, or 99 v/v% of the MV;
preferably, the MV is derived from a microbial strain in an intestine of a mammal;
preferably, the MV is derived from a genetically engineered microbial strain in an intestine of a mammal;
preferably, the bacterial strain with the immunostimulatory or immunomodulatory function is any one of following A1) to A6):
A1) a bacterial strain in which 16S rRNA has an identity of at least 95%, 96%, 97%, 98%, 98.5%, 98.65%, 99%, 99.5%, 99.6%, 99.7%, 99.8%, 99.9%, 99.99%, or 100% with a sequence shown in SEQ ID NO: 1;
A2) a bacterial strain with an accession number GDMCC No. 61121;
A3) a bacterial strain in which 16S rRNA has an identity of at least 95%, 96%, 97%, 98%, 98.5%, 98.65%, 99%, 99.5%, 99.6%, 99.7%, 99.8%, 99.9%, 99.99%, or 100% with 16S rRNA of the bacterial strain described in the A2);
A4) a bacterial strain in which a genome has an average nucleotide identity (ANI) of at least 87%, 90%, 95%, 96%, 97%, 98%, 99%, 99.5%, 99.6%, 99.7%, 99.8%, 99.9%, 99.99%, or 100% with a genome of a bacterial strain described in any of the A1) to the A3); and/or, a bacterial strain in which a genome has an alignment fraction of at least 60%, 70%, 80%, 90%, 95%, 96%, 97%, 98%, or 99% with a genome of a bacterial strain described in any of the A1) to the A3);
A5) a bacterial strain of a genus *Enterococcus;* and
A6) at least one bacterial strain of *Enterococcus faecalis* (*E. faecalis*), *Enterococcus faecium* (*E. faecium*), *Enterococcus asini (E. asini), Enterococcus avium (E. avium), Enterococcus canis* (*E. canis*), *Enterococcus casseliflavus (E. casseliflavus), Enterococcus cecorum (E. cecorum), Enterococcus columbae (E. columbae), Enterococcus dispar (E. dispar), Enterococcus durans (E. durans), Enterococcus flavescens (E. flavescens), Enterococcus gallinarum (E. gallinarum), Enterococcus gilvus (E. gilvus), Enterococcus haemoperoxidus (E. haemoperoxidus), Enterococcus hirae (E. hirae), Enterococcus malodoratus (E. malodoratus), Enterococcus moraviensis (E. moraviensis), Enterococcus mundtii (E. mundtii), Enterococcus pallens (E. pallens), Enterococcus phoeniculicola (E. phoeniculicola), Enterococcus porcinus (E. porcinus), Enterococcus pseudoavium (E. pseudoavium), Enterococcus raffinosus (E. raffinosus*), *Enterococcus ratti (E. ratti), Enterococcus saccharolyticus (E. saccharolyticus), Enterococcus sulfurous (E. sulfurous*), *Enterococcus villorum (E. villorum), Enterococcus devriesei (E. devriesei), Enterococcus hermanniensis (E. hermanniensis), Enterococcus saccharominimus (E. saccharominimus), Enterococcus aquimarinus (E. aquimarinus),* and *Enterococcus lactis;*
preferably, the MV is isolated from any bacterial strain of the A1) to the A6);
preferably, a mass percentage and/or volume percentage content of the microorganism-derived component in the composition is 1% to 99%, 2% to 90%, 5% to 80%, 10% to 70%, 20% to 60%, at least 45%, at least 50%, at least 55%, at least 60%, at least 70%, at least 80%, at least 90%, at least 95%, at least 98%, or at least 99%;
preferably, the composition further comprises at least one of an excipient, a diluent, and a carrier;
preferably, the composition further comprises an adjuvant;
preferably, the adjuvant comprises at least one of a lubricant, a wetting agent, a binder, an emulsifier, a suspension stabilizer, a solubilizing agent, a preservative, a sweetener, and a flavoring agent;
preferably, the composition is a solid preparation or a liquid preparation;
preferably, a dosage form of the composition is any one of a powder, a granule, a pulvis, a tablet, a capsule, a gel, a suspension, a drop, a drop pill, an injection, a suppository, an aerosol, an oral liquid, an ointment, an emulsion, and an irrigation solution; and
preferably, the composition further comprises a package insert.

2. The composition according to claim 1, further comprising a drug for combination therapy,
wherein preferably, the drug for combination therapy comprises an anti-tumor drug;
preferably, the anti-tumor drug comprises at least one of a chemotherapeutic drug, a photosensitizer, a photothermal therapy agent, and an immunotherapeutic drug;
preferably, the chemotherapeutic drug comprises at least one of paclitaxel, camptothecin, 5-fluorouracil, cisplatin, doxorubicin, mitomycin, and epirubicin;
preferably, the photosensitizer comprises at least one of BODIPY, chlorin, and rose bengal;
preferably, the photothermal therapy agent comprises at least one of a noble metal nanoparticle, an organic polymer, a carbon-based nanomaterial, a magnetic nanomaterial, and a semiconductor nanomaterial;
preferably, the immunotherapeutic drug comprises at least one of an immune cell therapy drug and an immune checkpoint inhibitor;
preferably, the immune cell therapy drug comprises at least one of a T cell therapy drug, a tumor-infiltrating lymphocyte therapy drug, and a natural killer (NK) cell therapy drug; and
preferably, the immune checkpoint inhibitor comprises an inhibitor targeting at least one of PD-1, PD-L1, PD-L2, CTLA-4, TIM-3, LAG-3, TIGIT, VISTA, BTLA, CD27, CD28, CD70, CD80, CD86, CD137, CD276, KIRs, TNFRSF4, GITR, GITRL, 4-1BBL, A2aR, VTCN1, IDO, and KLRA.

3. A use of the composition according to any one of claims 1 to 2 in preparation of a drug in at least one of following B1) to B4):
B1) preventing or treating a tumor or cancer;
B2) regulating or stimulating an immune system;
B3) regulating an intestine; and
B4) preventing and/or treating a disease mediated by an STING and/or NOD2 pathway,
wherein preferably, the tumor or cancer in the B1) comprises at least one of a solid tumor, a hematologic tumor, a soft tissue tumor, a glandular tumor, and a metastatic tumor;
preferably, the solid tumor comprises at least one of head and neck cancer, upper gastrointestinal cancer, lower gastrointestinal cancer, hepatobiliary system cancer, neuroendocrine tumor, thoracic cancer, bone tumor, soft tissue sarcoma, skin cancer, breast cancer, female reproductive system cancer, male reproductive system cancer, urinary system cancer, eye cancer, central nervous system cancer, and endocrine system cancer;
preferably, the hematologic tumor comprises a tumor in at least one of blood, lymph, and bone marrow;
preferably, the tumor or cancer comprises at least one of oral cancer, salivary gland cancer, nasopharyngeal cancer, oropharyngeal cancer, hypopharyngeal cancer, nasal cavity cancer, paranasal sinus cancer, laryngeal cancer, esophageal cancer, gastroesophageal junction cancer, gastric cancer, small intestine cancer, appendix cancer, colon cancer, rectal cancer, anal canal cancer, liver cancer, intrahepatic cholangiocarcinoma, gallbladder cancer, hilar cholangiocarcinoma, distal cholangiocarcinoma, ampullary cancer, pancreatic cancer, gastric neuroendocrine tumor, duodenal and ampullary neuroendocrine tumor, jejuno-ileal neuroendocrine tumor, appendiceal neuroendocrine tumor, colorectal neuroendocrine tumor, pancreatic neuroendocrine tumor, thymic carcinoma, lung cancer, malignant pleural mesothelioma, head and neck sarcomas, soft tissue sarcomas of the trunk and extremities, soft tissue sarcomas of abdominal and thoracic viscera, gastrointestinal stromal tumor, retroperitoneal soft tissue sarcoma, Merkel cell carcinoma, cutaneous malignant melanoma, vulvar cancer, vaginal cancer, cervical cancer, endometrial carcinoma, uterine carcinosarcoma, uterine sarcoma, ovarian cancer, fallopian tube cancer, primary peritoneal cancer, gestational trophoblastic neoplasia, penile cancer, prostate cancer, testicular cancer, kidney cancer, renal pelvis cancer, ureteral cancer, bladder cancer, urethral cancer, eyelid carcinoma, conjunctival carcinoma, conjunctival melanoma, uveal melanoma, retinoblastoma, lacrimal gland cancer, orbital sarcoma, ocular adnexal lymphoma, brain cancer, spinal cord tumor, differentiated thyroid cancer, anaplastic thyroid cancer, medullary thyroid cancer, parathyroid cancer, adrenocortical carcinoma, Hodgkin lymphoma, non-Hodgkin lymphoma, cutaneous lymphoma, plasma cell myeloma, and leukemia;
preferably, the tumor or cancer comprises a tumor or cancer caused by a bacterium or virus;
preferably, the bacterium or virus comprises at least one of hepatitis B virus, hepatitis C virus, human papillomavirus, Epstein-Barr (EB) virus, *Helicobacter pylori,* and *Fusobacterium nucleatum;*
preferably, the preventing or treating a tumor or cancer is achieved through at least one of following (a) to (j):
(a) inhibiting an increase in tumor volume;
(b) inhibiting an increase in tumor weight;
(c) inhibiting proliferation of a tumor cell;
(d) increasing a response rate of a tumor treatment;
(e) enhancing therapeutic efficacy of an immunosuppressive drug, comprising improving therapeutic efficacy of an anti-PD-1 antibody;
(f) preventing and/or inhibiting dissemination or metastasis of a tumor cell;
(g) reducing resistance to an anti-tumor drug;
(h) promoting a transcriptional activity of interferon-β (IFN-β);
(i) regulating or activating an immune system of a subject; and
(j) improving or activating an activity of the STING and/or NOD2 pathway;
preferably, the regulating or stimulating an immune system in the B2) comprises at least one of regulating or stimulating an innate immune response in a gastrointestinal tract, regulating or stimulating an innate immune response outside the gastrointestinal tract, inducing one or more beneficial immune effects in the gastrointestinal tract, and inducing one or more beneficial immune effects outside the gastrointestinal tract;
preferably, the regulating an intestine in the B3) comprises at least one of regulating an intestinal function and regulating a gut microbiota;
preferably, the regulating a gut microbiota comprises regulating at least one of species, richness, evenness, and diversity of the gut microbiota;
preferably, the regulating a gut microbiota comprises regulating a change in at least one of the species, the richness, the evenness, and the diversity of the gut microbiota that is induced by a disease and/or a drug;
preferably, the change in at least one of the species, the richness, the evenness, and the diversity of the gut microbiota that is induced by the disease and/or the drug comprises a change in at least one of the species, the richness, the evenness, and the diversity of the gut microbiota that is induced by any one of following D1) to D2):
D1) an infectious disease, a tumor or cancer, an inflammation, an allergic disease, or an autoimmune disease; and
D2) a drug for preventing and/or treating the D1);
preferably, the disease in the D1) is an intestinal disease;
preferably, the drug in the D2) is an anti-tumor drug;
preferably, the anti-tumor drug in the D2) comprises an immunotherapeutic drug;
preferably, the immunotherapeutic drug in the D2) comprises an immune checkpoint inhibitor;
preferably, the immune checkpoint inhibitor in the D2) comprises an inhibitor targeting at least one of PD-1, PD-L1, PD-L2, CTLA-4, TIM-3, LAG-3, TIGIT, VISTA, BTLA, CD27, CD28, CD70, CD80, CD86, CD137, CD276, KIRs, TNFRSF4, GITR, GITRL, 4-1BBL, A2aR, VTCN1, IDO, and KLRA; and
preferably, the disease mediated by the STING and/or NOD2 pathway comprises at least one of an infectious disease, a tumor or cancer, an inflammation, an allergic disease, and an autoimmune disease.

4. A use of the composition according to any one of claims 1 to 2 in at least one of following B1) to B4):
B1) preventing or treating a tumor or cancer;
B2) regulating or stimulating an immune system;
B3) regulating an intestine; and
B4) preventing and/or treating a disease mediated by an STING and/or NOD2 pathway,
wherein preferably, the tumor or cancer in the B1) comprises at least one of a solid tumor, a hematologic tumor, a soft tissue tumor, a glandular tumor, and a metastatic tumor;
preferably, the solid tumor comprises at least one of head and neck cancer, upper gastrointestinal cancer, lower gastrointestinal cancer, hepatobiliary system cancer, neuroendocrine tumor, thoracic cancer, bone tumor, soft tissue sarcoma, skin cancer, breast cancer, female reproductive system cancer, male reproductive system cancer, urinary system cancer, eye cancer, central nervous system cancer, and endocrine system cancer;
preferably, the hematologic tumor comprises a tumor in at least one of blood, lymph, and bone marrow;
preferably, the tumor or cancer comprises at least one of oral cancer, salivary gland cancer, nasopharyngeal cancer, oropharyngeal cancer, hypopharyngeal cancer, nasal cavity cancer, paranasal sinus cancer, laryngeal cancer, esophageal cancer, gastroesophageal junction cancer, gastric cancer, small intestine cancer, appendix cancer, colon cancer, rectal cancer, anal canal cancer, liver cancer, intrahepatic cholangiocarcinoma, gallbladder cancer, hilar cholangiocarcinoma, distal cholangiocarcinoma, ampullary cancer, pancreatic cancer, gastric neuroendocrine tumor, duodenal and ampullary neuroendocrine tumor, jejuno-ileal neuroendocrine tumor, appendiceal neuroendocrine tumor, colorectal neuroendocrine tumor, pancreatic neuroendocrine tumor, thymic carcinoma, lung cancer, malignant pleural mesothelioma, head and neck sarcomas, soft tissue sarcomas of the trunk and extremities, soft tissue sarcomas of abdominal and thoracic viscera, gastrointestinal stromal tumor, retroperitoneal soft tissue sarcoma, Merkel cell carcinoma, cutaneous malignant melanoma, vulvar cancer, vaginal cancer, cervical cancer, endometrial carcinoma, uterine carcinosarcoma, uterine sarcoma, ovarian cancer, fallopian tube cancer, primary peritoneal cancer, gestational trophoblastic neoplasia, penile cancer, prostate cancer, testicular cancer, kidney cancer, renal pelvis cancer, ureteral cancer, bladder cancer, urethral cancer, eyelid carcinoma, conjunctival carcinoma, conjunctival melanoma, uveal melanoma, retinoblastoma, lacrimal gland cancer, orbital sarcoma, ocular adnexal lymphoma, brain cancer, spinal cord tumor, differentiated thyroid cancer, anaplastic thyroid cancer, medullary thyroid cancer, parathyroid cancer, adrenocortical carcinoma, Hodgkin lymphoma, non-Hodgkin lymphoma, cutaneous lymphoma, plasma cell myeloma, and leukemia;
preferably, the tumor or cancer comprises a tumor or cancer caused by a bacterium or virus;
preferably, the bacterium or virus comprises at least one of hepatitis B virus, hepatitis C virus, human papillomavirus, EB virus, *Helicobacter pylori,* and *Fusobacterium nucleatum;*
preferably, the preventing or treating a tumor or cancer is achieved through at least one of following (a) to (j):
(a) inhibiting an increase in tumor volume;
(b) inhibiting an increase in tumor weight;
(c) inhibiting proliferation of a tumor cell;
(d) increasing a response rate of a tumor treatment;
(e) enhancing therapeutic efficacy of an immunosuppressive drug, comprising improving therapeutic efficacy of an anti-PD-1 antibody;
(f) preventing and/or inhibiting dissemination or metastasis of a tumor cell;
(g) reducing resistance to an anti-tumor drug;
(h) promoting a transcriptional activity of IFN-β;
(i) regulating or activating an immune system of a subject; and
(j) improving or activating an activity of the STING and/or NOD2 pathway;
preferably, the regulating or stimulating an immune system in the B2) comprises at least one of regulating or stimulating an innate immune response in a gastrointestinal tract, regulating or stimulating an innate immune response outside the gastrointestinal tract, inducing one or more beneficial immune effects in the gastrointestinal tract, and inducing one or more beneficial immune effects outside the gastrointestinal tract;
preferably, the regulating an intestine in the B3) comprises at least one of regulating an intestinal function and regulating a gut microbiota;
preferably, the regulating a gut microbiota comprises regulating at least one of species, richness, evenness, and diversity of the gut microbiota;
preferably, the regulating a gut microbiota comprises regulating a change in at least one of the species, the richness, the evenness, and the diversity of the gut microbiota that is induced by a disease and/or a drug;
preferably, the change in at least one of the species, the richness, the evenness, and the diversity of the gut microbiota that is induced by the disease and/or the drug comprises a change in at least one of the species, the richness, the evenness, and the diversity of the gut microbiota that is induced by any one of following D1) to D2):
D1) an infectious disease, a tumor or cancer, an inflammation, an allergic disease, or an autoimmune disease; and
D2) a drug for preventing and/or treating the D1);
preferably, the disease in the D1) is an intestinal disease;
preferably, the drug in the D2) is an anti-tumor drug;
preferably, the anti-tumor drug in the D2) comprises an immunotherapeutic drug;
preferably, the immunotherapeutic drug in the D2) comprises an immune checkpoint inhibitor;
preferably, the immune checkpoint inhibitor in the D2) comprises an inhibitor targeting at least one of PD-1, PD-L1, PD-L2, CTLA-4, TIM-3, LAG-3, TIGIT, VISTA, BTLA, CD27, CD28, CD70, CD80, CD86, CD137, CD276, KIRs, TNFRSF4, GITR, GITRL, 4-1BBL, A2aR, VTCN1, IDO, and KLRA; and
preferably, the disease mediated by the STING and/or NOD2 pathway comprises at least one of an infectious disease, a tumor or cancer, an inflammation, an allergic disease, and an autoimmune disease.

5. A method for preventing or treating a tumor or cancer, and/or regulating or activating an immune system of a subject, and/or regulating an intestine, and/or preventing and/or treating a disease mediated by an STING and/or NOD2 pathway, comprising administering an effective dose of the composition according to any one of claims 1 to 2 to the subject,
wherein preferably, the tumor or cancer comprises at least one of a solid tumor, a hematologic tumor, a soft tissue tumor, a glandular tumor, and a metastatic tumor;
preferably, the solid tumor comprises at least one of head and neck cancer, upper gastrointestinal cancer, lower gastrointestinal cancer, hepatobiliary system cancer, neuroendocrine tumor, thoracic cancer, bone tumor, soft tissue sarcoma, skin cancer, breast cancer, female reproductive system cancer, male reproductive system cancer, urinary system cancer, eye cancer, central nervous system cancer, and endocrine system cancer;
preferably, the hematologic tumor comprises a tumor in at least one of blood, lymph, and bone marrow;
preferably, the tumor or cancer comprises at least one of oral cancer, salivary gland cancer, nasopharyngeal cancer, oropharyngeal cancer, hypopharyngeal cancer, nasal cavity cancer, paranasal sinus cancer, laryngeal cancer, esophageal cancer, gastroesophageal junction cancer, gastric cancer, small intestine cancer, appendix cancer, colon cancer, rectal cancer, anal canal cancer, liver cancer, intrahepatic cholangiocarcinoma, gallbladder cancer, hilar cholangiocarcinoma, distal cholangiocarcinoma, ampullary cancer, pancreatic cancer, gastric neuroendocrine tumor, duodenal and ampullary neuroendocrine tumor, jejuno-ileal neuroendocrine tumor, appendiceal neuroendocrine tumor, colorectal neuroendocrine tumor, pancreatic neuroendocrine tumor, thymic carcinoma, lung cancer, malignant pleural mesothelioma, head and neck sarcomas, soft tissue sarcomas of the trunk and extremities, soft tissue sarcomas of abdominal and thoracic viscera, gastrointestinal stromal tumor, retroperitoneal soft tissue sarcoma, Merkel cell carcinoma, cutaneous malignant melanoma, vulvar cancer, vaginal cancer, cervical cancer, endometrial carcinoma, uterine carcinosarcoma, uterine sarcoma, ovarian cancer, fallopian tube cancer, primary peritoneal cancer, gestational trophoblastic neoplasia, penile cancer, prostate cancer, testicular cancer, kidney cancer, renal pelvis cancer, ureteral cancer, bladder cancer, urethral cancer, eyelid carcinoma, conjunctival carcinoma, conjunctival melanoma, uveal melanoma, retinoblastoma, lacrimal gland cancer, orbital sarcoma, ocular adnexal lymphoma, brain cancer, spinal cord tumor, differentiated thyroid cancer, anaplastic thyroid cancer, medullary thyroid cancer, parathyroid cancer, adrenocortical carcinoma, Hodgkin lymphoma, non-Hodgkin lymphoma, cutaneous lymphoma, plasma cell myeloma, and leukemia;
preferably, the tumor or cancer comprises a tumor or cancer caused by a bacterium or virus;
preferably, the bacterium or virus comprises at least one of hepatitis B virus, hepatitis C virus, human papillomavirus, EB virus, *Helicobacter pylori,* and *Fusobacterium nucleatum;*
preferably, the preventing or treating a tumor or cancer is achieved through at least one of following (a) to (j):
(a) inhibiting an increase in tumor volume;
(b) inhibiting an increase in tumor weight;
(c) inhibiting proliferation of a tumor cell;
(d) increasing a response rate of a tumor treatment;
(e) enhancing therapeutic efficacy of an immunosuppressive drug, comprising improving therapeutic efficacy of an anti-PD-1 antibody;
(f) preventing and/or inhibiting dissemination or metastasis of a tumor cell;
(g) reducing resistance to an anti-tumor drug;
(h) promoting a transcriptional activity of IFN-β;
(i) regulating or activating an immune system of a subject; and
(j) improving or activating an activity of the STING and/or NOD2 pathway;
preferably, the regulating or stimulating an immune system in the B2) comprises at least one of regulating or stimulating an innate immune response in a gastrointestinal tract, regulating or stimulating an innate immune response outside the gastrointestinal tract, inducing one or more beneficial immune effects in the gastrointestinal tract, and inducing one or more beneficial immune effects outside the gastrointestinal tract;
preferably, the regulating an intestine comprises at least one of regulating an intestinal function and regulating a gut microbiota;
preferably, the regulating a gut microbiota comprises regulating at least one of species, richness, evenness, and diversity of the gut microbiota;
preferably, the regulating a gut microbiota comprises regulating a change in at least one of the species, the richness, the evenness, and the diversity of the gut microbiota that is induced by a disease and/or a drug;
preferably, the change in at least one of the species, the richness, the evenness, and the diversity of the gut microbiota that is induced by the disease and/or the drug comprises a change in at least one of the species, the richness, the evenness, and the diversity of the gut microbiota that is induced by any one of following D1) to D2):
D1) an infectious disease, a tumor or cancer, an inflammation, an allergic disease, or an autoimmune disease; and
D2) a drug for preventing and/or treating the D1);
preferably, the disease in the D1) is an intestinal disease;
preferably, the drug in the D2) is an anti-tumor drug;
preferably, the anti-tumor drug in the D2) comprises an immunotherapeutic drug;
preferably, the immunotherapeutic drug in the D2) comprises an immune checkpoint inhibitor;
preferably, the immune checkpoint inhibitor in the D2) comprises an inhibitor targeting at least one of PD-1, PD-L1, PD-L2, CTLA-4, TIM-3, LAG-3, TIGIT, VISTA, BTLA, CD27, CD28, CD70, CD80, CD86, CD137, CD276, KIRs, TNFRSF4, GITR, GITRL, 4-1BBL, A2aR, VTCN1, IDO, and KLRA; and
preferably, the disease mediated by the STING and/or NOD2 pathway comprises at least one of an infectious disease, a tumor or cancer, an inflammation, an allergic disease, and an autoimmune disease.

6. The method according to claim 5, wherein the composition is administered at least once daily;
preferably, when the composition is administered twice or more daily, the composition is administered at a same dose;
preferably, when the composition is administered twice or more daily, the composition is administered at different doses; and
preferably, a route for the administering comprises at least one of oral administration, sublingual administration, nasal administration, rectal administration, inhalation administration, transdermal administration, intraperitoneal injection, subcutaneous injection, and intramuscular injection.

7. A use of C1 and C2 in preparation of an anti-tumor drug, wherein an anti-tumor activity of the anti-tumor drug comprises prevention or treatment of at least one tumor of kidney cancer, liver cancer, and fibrosarcoma;
the C1 comprises at least one of a bacterial strain of a genus *Enterococcus,* a metabolite of the bacterial strain of the genus *Enterococcus,* a culture of the bacterial strain of the genus *Enterococcus,* and a fermentation supernatant of the bacterial strain of the genus *Enterococcus,* and the C2 comprises an immune checkpoint inhibitor;
preferably, the bacterial strain of the genus *Enterococcus* comprises at least one of *E*. *faecalis, E. faecium, E. asini, E. avium, E. canis, E. casseliflavus, E. cecorum, E. columbae, E. dispar, E. durans, E. flavescens, E. gallinarum, E. gilvus, E. haemoperoxidus, E. hirae, E. malodoratus, E. moraviensis, E. mundtii, E. pallens, E. phoeniculicola, E. porcinus, E. pseudoavium, E. raffinosus, E. ratti, E. saccharolyticus, E. sulfurous, E. villorum, E. devriesei, E. hermanniensis, E. saccharominimus, E. aquimarinus,* and *Enterococcus lactis;*
preferably, the bacterial strain of the genus *Enterococcus* is any one of following A1) to A4):
A1) a bacterial strain in which 16S rRNA has an identity of at least 95%, 96%, 97%, 98%, 98.5%, 98.65%, 99%, 99.5%, 99.6%, 99.7%, 99.8%, 99.9%, 99.99%, or 100% with a sequence shown in SEQ ID NO: 1;
A2) a bacterial strain with an accession number GDMCC No. 61121;
A3) a bacterial strain in which 16S rRNA has an identity of at least 95%, 96%, 97%, 98%, 98.5%, 98.65%, 99%, 99.5%, 99.6%, 99.7%, 99.8%, 99.9%, 99.99%, or 100% with 16S rRNA of the bacterial strain described in the A2); and
A4) a bacterial strain in which a genome has an average nucleotide identity (ANI) of at least 87%, 90%, 95%, 96%, 97%, 98%, 99%, 99.5%, 99.6%, 99.7%, 99.8%, 99.9%, 99.99%, or 100% with a genome of a bacterial strain described in any of the A1) to the A3); and/or, a bacterial strain in which a genome has an alignment fraction of at least 60%, 70%, 80%, 90%, 95%, 96%, 97%, 98%, or 99% with a genome of a bacterial strain described in any of the A1) to the A3);
preferably, the immune checkpoint inhibitor comprises an inhibitor targeting at least one of PD-1, PD-L1, PD-L2, CTLA-4, TIM-3, LAG-3, TIGIT, VISTA, BTLA, CD27, CD28, CD70, CD80, CD86, CD137, CD276, KIRs, TNFRSF4, GITR, GITRL, 4-1BBL, A2aR, VTCN1, IDO, and KLRA; and
preferably, the immune checkpoint inhibitor comprises an anti-PD-1 antibody.

8. A use of C1 and C2 in prevention or treatment of at least one tumor of kidney cancer, liver cancer, and fibrosarcoma,
wherein the C1 comprises at least one of a bacterial strain of a genus *Enterococcus, a* metabolite of the bacterial strain of the genus *Enterococcus,* a culture of the bacterial strain of the genus *Enterococcus,* and a fermentation supernatant of the bacterial strain of the genus *Enterococcus,* and the C2 comprises an immune checkpoint inhibitor;
preferably, the bacterial strain of the genus *Enterococcus* comprises at least one of *E*. *faecalis, E. faecium, E. asini, E. avium, E. canis, E. casseliflavus, E. cecorum, E. columbae, E. dispar, E. durans, E. flavescens, E. gallinarum, E. gilvus, E. haemoperoxidus, E. hirae, E. malodoratus, E. moraviensis, E. mundtii, E. pallens, E. phoeniculicola, E. porcinus, E. pseudoavium, E. raffinosus, E. ratti, E. saccharolyticus, E. sulfurous, E. villorum, E. devriesei, E. hermanniensis, E. saccharominimus, E. aquimarinus,* and *Enterococcus lactis;*
preferably, the bacterial strain of the genus *Enterococcus* is any one of following A1) to A4):
A1) a bacterial strain in which 16S rRNA has an identity of at least 95%, 96%, 97%, 98%, 98.5%, 98.65%, 99%, 99.5%, 99.6%, 99.7%, 99.8%, 99.9%, 99.99%, or 100% with a sequence shown in SEQ ID NO: 1;
A2) a bacterial strain with an accession number GDMCC No. 61121;
A3) a bacterial strain in which 16S rRNA has an identity of at least 95%, 96%, 97%, 98%, 98.5%, 98.65%, 99%, 99.5%, 99.6%, 99.7%, 99.8%, 99.9%, 99.99%, or 100% with 16S rRNA of the bacterial strain described in the A2); and
A4) a bacterial strain in which a genome has an average nucleotide identity (ANI) of at least 87%, 90%, 95%, 96%, 97%, 98%, 99%, 99.5%, 99.6%, 99.7%, 99.8%, 99.9%, 99.99%, or 100% with a genome of a bacterial strain described in any of the A1) to the A3); and/or, a bacterial strain in which a genome has an alignment fraction of at least 60%, 70%, 80%, 90%, 95%, 96%, 97%, 98%, or 99% with a genome of a bacterial strain described in any of the A1) to the A3);
preferably, the immune checkpoint inhibitor comprises an inhibitor targeting at least one of PD-1, PD-L1, PD-L2, CTLA-4, TIM-3, LAG-3, TIGIT, VISTA, BTLA, CD27, CD28, CD70, CD80, CD86, CD137, CD276, KIRs, TNFRSF4, GITR, GITRL, 4-1BBL, A2aR, VTCN1, IDO, and KLRA; and
preferably, the immune checkpoint inhibitor comprises an anti-PD-1 antibody.

9. A method for preventing or treating at least one tumor of kidney cancer, liver cancer, and fibrosarcoma, comprising administering an effective dose of C1 and C2 to a subject,
wherein the C1 comprises at least one of a bacterial strain of a genus *Enterococcus, a* metabolite of the bacterial strain of the genus *Enterococcus,* a culture of the bacterial strain of the genus *Enterococcus,* and a fermentation supernatant of the bacterial strain of the genus *Enterococcus,* and the C2 comprises an immune checkpoint inhibitor;
preferably, the bacterial strain of the genus *Enterococcus* comprises at least one of *E*. *faecalis, E. faecium, E. asini, E. avium, E. canis, E. casseliflavus, E. cecorum, E. columbae, E. dispar, E. durans, E. flavescens, E. gallinarum, E. gilvus, E. haemoperoxidus, E. hirae, E. malodoratus, E. moraviensis, E. mundtii, E. pallens, E. phoeniculicola, E. porcinus, E. pseudoavium, E. raffinosus, E. ratti, E. saccharolyticus, E. sulfurous, E. villorum, E. devriesei, E. hermanniensis, E. saccharominimus, E. aquimarinus,* and *Enterococcus lactis;*
preferably, the bacterial strain of the genus *Enterococcus* is any one of following A1) to A4):
A1) a bacterial strain in which 16S rRNA has an identity of at least 95%, 96%, 97%, 98%, 98.5%, 98.65%, 99%, 99.5%, 99.6%, 99.7%, 99.8%, 99.9%, 99.99%, or 100% with a sequence shown in SEQ ID NO: 1;
A2) a bacterial strain with an accession number GDMCC No. 61121;
A3) a bacterial strain in which 16S rRNA has an identity of at least 95%, 96%, 97%, 98%, 98.5%, 98.65%, 99%, 99.5%, 99.6%, 99.7%, 99.8%, 99.9%, 99.99%, or 100% with 16S rRNA of the bacterial strain described in the A2); and
A4) a bacterial strain in which a genome has an average nucleotide identity (ANI) of at least 87%, 90%, 95%, 96%, 97%, 98%, 99%, 99.5%, 99.6%, 99.7%, 99.8%, 99.9%, 99.99%, or 100% with a genome of a bacterial strain described in any of the A1) to the A3); and/or, a bacterial strain in which a genome has an alignment fraction of at least 60%, 70%, 80%, 90%, 95%, 96%, 97%, 98%, or 99% with a genome of a bacterial strain described in any of the A1) to the A3);
preferably, the immune checkpoint inhibitor comprises an inhibitor targeting at least one of PD-1, PD-L1, PD-L2, CTLA-4, TIM-3, LAG-3, TIGIT, VISTA, BTLA, CD27, CD28, CD70, CD80, CD86, CD137, CD276, KIRs, TNFRSF4, GITR, GITRL, 4-1BBL, A2aR, VTCN1, IDO, and KLRA; and
preferably, the immune checkpoint inhibitor comprises an anti-PD-1 antibody.

10. The method according to claim 9, wherein the C1 and the C2 are administered at least once daily;
preferably, the C1 and the C2 are administered simultaneously, separately, or sequentially;
preferably, when the C1 or the C2 is administered twice or more daily, the C1 or the C2 is administered at a same dose;
preferably, when the C1 or the C2 is administered twice or more daily, the C1 or the C2 is administered at different doses; and
preferably, a route for the administering comprises at least one of oral administration, sublingual administration, nasal administration, rectal administration, inhalation administration, transdermal administration, intragastric administration, intraperitoneal injection, subcutaneous injection, and intramuscular injection.
